(19) **Europäisches Patentamt / European Patent Office / Office européen des brevets**

(11) **EP 1 591 058 B1**

(12) **EUROPEAN PATENT SPECIFICATION**

(45) Date of publication and mention
of the grant of the patent:
**17.09.2014 Bulletin 2014/38**

(51) Int Cl.:
*A61B 1/00* (2006.01)  *A61B 1/04* (2006.01)
*A61B 19/00* (2006.01)

(21) Application number: **04708041.1**

(22) Date of filing: **04.02.2004**

(86) International application number:
**PCT/JP2004/001105**

(87) International publication number:
**WO 2004/069043 (19.08.2004 Gazette 2004/34)**

(54) **MEDICAL APPARATUS GUIDING SYSTEM**

FÜHRUNGSSYSTEM FÜR MEDIZINPRODUKTE

SYSTEME DE GUIDAGE DE DISPOSITIF MEDICAL

(84) Designated Contracting States:
**DE FR GB**

(30) Priority: **04.02.2003 JP 2003027476**
**26.01.2004 JP 2004017606**

(43) Date of publication of application:
**02.11.2005 Bulletin 2005/44**

(73) Proprietor: **Olympus Corporation**
**Tokyo 151-0072 (JP)**

(72) Inventors:
• **UCHIYAMA, Akio**
**Hachioji-shi**
**Tokyo 192-8512 (JP)**
• **KAWANO, Hironao**
**Hachioji-shi**
**Tokyo 192-8512 (JP)**
• **YOKOI, Takeshi**
**Hachioji-shi**
**Tokyo 192-8512 (JP)**

• **TAKIZAWA, Hironobu**
**Hachioji-shi**
**Tokyo 192-8512 (JP)**
• **ARAI, Kenichi**
**Hachioji-shi**
**Tokyo 192-8512 (JP)**
• **ISHIYAMA, Kazushi**
**Hachioji-shi**
**Tokyo 192-8512 (JP)**
• **SENDOH, Masahiko**
**Hachioji-shi**
**Tokyo 192-8512 (JP)**

(74) Representative: **von Hellfeld, Axel**
**Wuesthoff & Wuesthoff**
**Patent- und Rechtsanwälte**
**Schweigerstrasse 2**
**81541 München (DE)**

(56) References cited:
**JP-A- 4 008 341      JP-A- 8 322 786**
**JP-A- 2001 179 700**

EP 1 591 058 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a medical apparatus guiding system which advances while rotating, and guides a medical apparatus main body inserted in the body cavity by using magnetic means or the like.

Background Art

As a first conventional art, Japanese Patent No. 3017770 discloses a medical apparatus for magnetic guide in a subject.

According to the first conventional art, the medical apparatus comprises a guided portion which is magnetically guided in at least one part of an inserting portion that is inserted in the subject. Further, the medical apparatus comprises magnetic force generating means arranged to the outside of the subject and moving means. The movement of the guided portion matches that of the medical apparatus in one guided direction by the magnetic force generating means. However, in the direction which is not controlled for matching the movements between them, the moving means moves the magnetic force generating means.

Here, Japanese Patent No. 3017770 further discloses a method for magnetically guiding a capsule endoscope or a normal endoscope inserting portion. Furthermore, it discloses a method for vibrating the endoscope inserting portion by an alternating magnetic field or for guiding the capsule endoscope while rotating thereof.

As a second conventional art, Japanese Unexamined Patent Application Publication No. 2001-179700 discloses a medical apparatus comprising a magnetic field generating unit which generates a rotating magnetic field, and a robot main body which obtains thrust by receiving the rotating magnetic field and rotating it, wherein the rotating magnetic field surface can be changed in the predetermined direction on the three-dimensional space.

Further, Japanese Unexamined Patent Application Publication No. 2001-179700 discloses a thrust generating unit which is formed by arranging, to the robot main body, mechanical means such as a spiral and a screw suitable to the advance in the fluid and a thrust generating unit which is formed by arranging a drill unit at the front edge and the rear edge of the robot main body so as to enable the movement thereof if a solid material or gel material exists in the advancing direction.

The above-mentioned conventional art has such a drawback that a magnetic guiding for the capsule endoscope or medical apparatus main body inserted in the body cavity once stops and then the capsule endoscope or medical apparatus main body is not smoothly magnetically guided thereafter.

Further, the smooth guiding is not possible because of non-disclosure of means which prevents the instruction of the advancing direction excessively different from the current direction of the capsule endoscope or medical apparatus main body inserted in the body cavity.

Document JP 8 322786 A discloses a diagnosing / treating apparatus for the inside of an organism. The apparatus is provided with a tubular scope having a plurality of cells, an optical fiber connected to each of cells, a light supply device to supply light to the optical fiber, and a controller to control the quantity of light to be supplied of the light supply device using an address signal and an ON/OFF signal. A scope can be bent in an arbitrary direction and a diagnosing/treating device such as CCD camera or washing device is provided at the tip part thereof. The scope can be bent in a desired direction by controlling the expansion and shrinking of the plurality of cells.

Document JP 4 008341 A discloses an inserting device comprising magnetic field detecting means, which detects a magnetic field generated by a magnetic field generating means and a position where it is provided to an insertion part, in the outside of a testee body. After the insertion part is inserted to a certain degree into the testee body such as the colon or the like, position detection of a point end constitutional part is indicated by an operating means. Then, a hole sensor is scanned in a horizontal surface by controlling a motor drive circuit by a controller. An output of the hole sensor is obtained in each position, and a position, where the output of this hole sensor is obtained maximum, is obtained as a position of the point end constitutional part. After the position of the point end constitutional part is thus detected, the insertion part, magnetically guided, is inserted further into the testee body. That is, a magnetic field is generated from a magnetic force generating part through the controller by a command of the operating means.

Therefore, it is an object of the present invention to provide a medical apparatus guiding system which simultaneously rotates and advances by means of magnetism and thus smoothly guides a medical apparatus main body inserted in the body cavity by using magnetic means.

Disclosure of Invention

According to the present invention, a medical apparatus guiding system includes the features of claim 1, and a control method includes the features of claim 27.

Consequently, the medical apparatus main body is smoothly guided.

Brief Description of the Drawings

Figs. 1 to 13 relate to the first embodiment of the present disclosure, Fig. 1 is a block diagram showing the inner structure of units in a capsule medical apparatus guiding system according to the first embodiment;
Fig. 2 is a diagram showing the entire structure of the capsule medical apparatus guiding system;
Figs. 3A and 3B are a side view and a front view of a capsule main body, respectively;
Fig. 4A is a perspective view showing the structure of an operation input unit;
Fig. 4B is a side view showing the structure of an operation input unit according to one modification;
Fig. 4C is a diagram showing a foot switch in place of a stick shown in Fig. 4A;
Fig. 5A is a diagram showing a coordinate system of a normal-vector direction of the rotating surface of a rotating magnetic filed;
Fig. 5B is a diagram showing the advancing direction of a capsule main body upon inclining a joystick;
Fig. 5C is a diagram showing the rotating direction upon inclining a stick forward and backward;
Fig. 6A is a diagram showing the structure excluding a function button shown in Fig. 4B according to another modification;
Fig. 6B is a diagram showing the advancing direction of a capsule main body upon inclining the joystick;
Fig. 6C is an explanatory diagram showing the capsule main body and the advancing direction of the capsule main body on the three-dimensional coordinate system;
Figs. 7A and 7B are diagrams showing the time change of a magnetic field component upon applying the rotating magnetic field and stopping it, respectively;
Fig. 8 is an explanatory diagram showing the change state of the rotating magnetic field upon applying the rotating magnetic field;
Fig. 9 is a flowchart showing a part of processing for setting and displaying the up direction of the image display in the specific direction upon rotating the capsule main body;
Fig. 10 is a flowchart showing the remaining processing contents shown in Fig. 9;
Fig. 11 is an explanatory diagram of the operations shown in Figs. 9 and 10;
Fig. 12 is an explanatory diagram of an operating method using GUI;
Fig. 13 is a flowchart for explaining the operation of a control device in accordance with the time passage;
Figs. 14 to 18 relate to the second embodiment of the present disclosure, according to the invention, Fig. 14 is a block diagram showing the inner structure of units in a capsule medical apparatus guiding system according to the second embodiment;
Fig. 15 is a side view of a capsule main body;
Fig. 16 is a diagram showing a display example of a display device;
Fig. 17 is an explanatory diagram of the operation; and
Figs. 18A and 18B are side view and a front view showing the structure of a capsule medical apparatus according to a modification.

Best Mode for Carrying Out the Invention

Hereinbelow, embodiments of the present disclosure will be described with reference to the drawings.

(First embodiment)

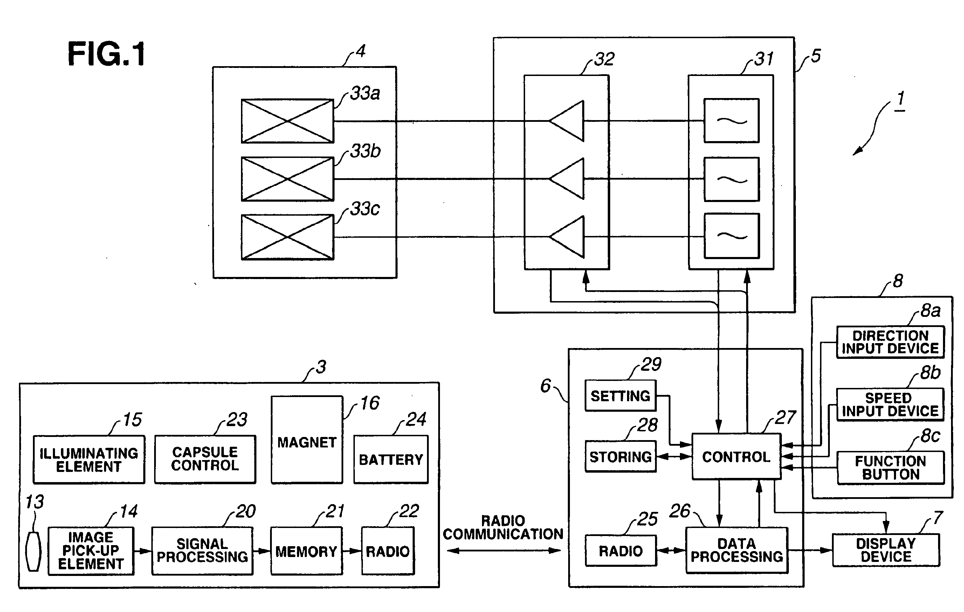

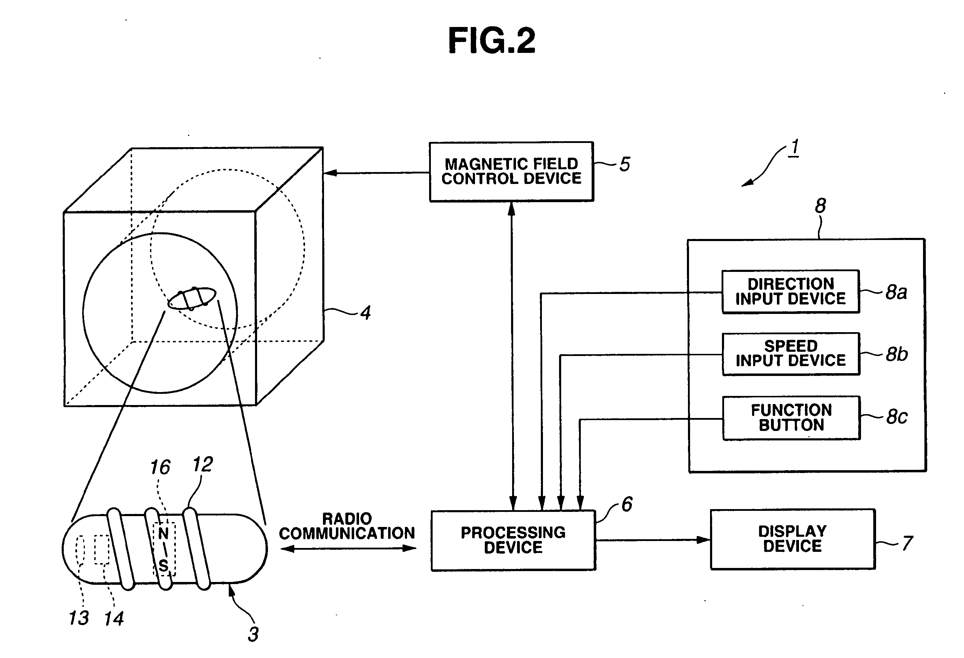

The first embodiment of the present disclosure will be described with reference to Figs. 1 to 13.
Referring to Figs. 1 and 2, a capsule medical apparatus guiding system 1 according to the first embodiment of the present disclosure comprises: a capsule medical apparatus main body 3 (hereinafter, abbreviated to a capsule main body) which is inserted in the body cavity of a patient (not shown) and which functions as a capsule endoscope that picks up an image of the body cavity; a rotating magnetic field generating device 4 which is extracorporeally arranged around the patient and which applies a rotating magnetic field to the capsule main body 3; a magnetic field control device (or power supply control device) 5 which controls the operation for supplying driving current that generates the rotating magnetic field in the rotating magnetic field generating device 4; a processing device 6 which is extracorporeally arranged to the patient, performs the processing for radio communication with the capsule main body 3, and controls the magnetic field control device 5 so as to control the direction and the level of the rotating magnetic field applied to the capsule main body 3; a display device 7 which is connected to the processing device 6 and which displays an image picked up by the capsule main body 3; and an operation input unit 8 for instructing and inputting an instructing signal corresponding to the operation which is performed by an operator, including a direction input portion 8a which generates the instructing signal in the magnetic field direction, a speed input unit 8b which generates the instructing signal of the rotating magnetic field of a rotating frequency corresponding to the operation, and a function button 8c which generates the instructing signal corresponding to a set function such as the generation of the de-centered rotating magnetic field corresponding to the operation.

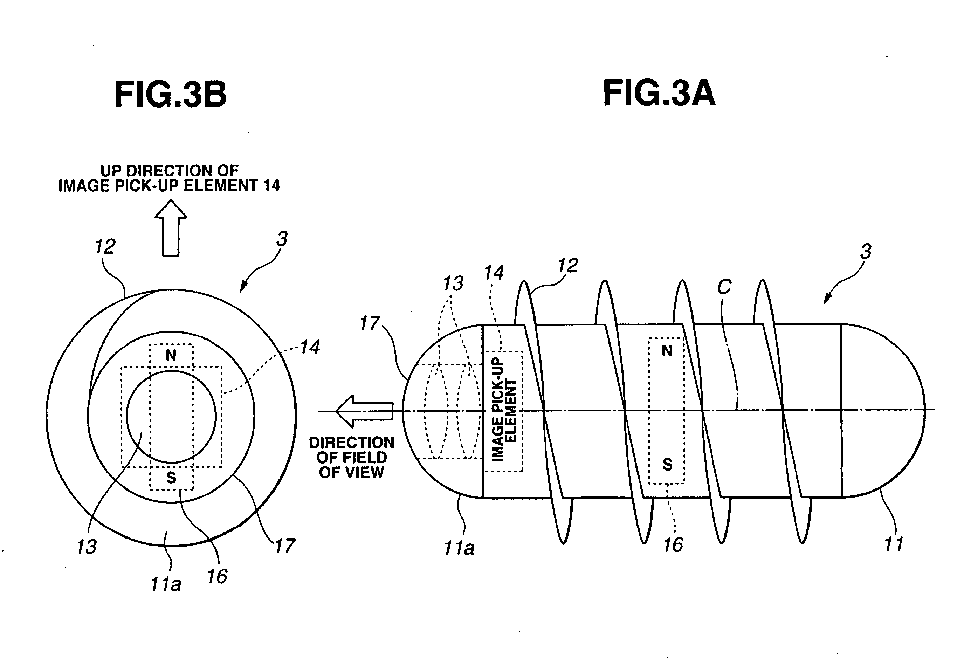

Referring to Fig. 3, the capsule main body 3 has a spiral projection (or a screw portion) 12 as a thrust generating structure unit which generates the thrust by rotating a capsule exterior container 11 on the outer peripheral surface thereof. Further, the inner portion sealed by the exterior container 11 has an objective optical system 13, an image pick-up element 14 which is arranged to the image forming position of the objective optical system 13, an illuminating element 15 for illumination for the purpose of the image pick-up operation (refer to Fig. 1), and a magnet 16.

Referring to Fig. 3, the objective optical system 13 is arranged in a semispherical and transparent edge cover 11a in the exterior container 11 so that the optical axis of the objective optical system 13 matches a center axis C of the cylindrical capsule main body 3. Referring to Fig. 3B, the center portion of the edge cover 11a becomes an observing window 17. Although not shown in Fig. 3, the illuminating element 15 is arranged around the objective optical system 13.

In this case, the field-of-view direction of the objective optical system 13 becomes the optical axis of the objective optical system 13, that is, the direction along the center axis C of the cylindrical capsule main body 3.

Referring to Fig. 3, the magnet 16 arranged near the center of the capsule main body 3 in the longitudinal direction thereof has the N pole and S pole in the direction orthogonal to the center axis C. In this case, the center of the magnet 16 is arranged matching the barycentric position of the capsule main body 3. Upon applying the magnetic field from the outside, the center of the magnetic force which exerts on the magnet 16 becomes the barycentric position of the capsule main body 3, and the capsule main body 3 smoothly advances with the magnetic force.

Referring to Fig. 3B, the magnet 16 is arranged matching the specific arranging direction of the image pick-up element 14.

That is, the up direction upon displaying the image picked up by the image pick-up element 14 is set in the direction to the N pole from the S pole of the magnet 16.

By applying the rotating magnetic field to the capsule main body 3 by the rotating magnetic field generating device 4, the magnet 16 is magnetically rotated. Then, the capsule main body 3 which fixes the magnet 16 therein is rotated together with the magnet 16. In this case, the spiral projection 12 arranged to the outer peripheral surface of the capsule main body 3 is in contact with the inner wall of the body cavity and is rotated to advance the capsule main body 3.

As mentioned above, upon controlling the capsule main body 3 including the magnet 16 by the external magnetic field, it is known which direction is the up direction of the image picked up by the capsule main body 3 from the direction of the external magnetic field.

Referring to Fig. 1, the capsule main body 3 comprises: the objective optical system 13; the image pick-up element 14; the magnet 16; a signal processing circuit 20 which performs the signal processing of the signal picked up by the image pick-up element 14; a memory 21 which temporarily stores a digital video signal generated by the signal processing circuit 20; a radio circuit 22 which modulates the video signal read from the memory 21 by a high-frequency signal, converts the modulated into a signal suitable for radio transmission, and demodulates a control signal transmitted from the processing device 6; a capsule control circuit 23 which controls the capsule main body 3 including the signal processing circuit 20 and the like; and a battery 24 which supplies power for operation to an electric system in the capsule main body 3 including the signal processing circuit 20 and the like.

The processing device 6 for radio communication with the capsule main body 3 comprises: a radio circuit 25 which communicates with the radio circuit 22 by radio waves; a data processing circuit 26 which is connected to the radio circuit 25 and performs the data processing including image display operation of image data transmitted from the capsule main body 3; a control circuit 27 which controls the data processing circuit 26 and the power supply control device 5; a storing circuit 28 which stores the state of the rotating magnetic field generated by the rotating magnetic field generating device 4 via the power supply control device 5, specifically, information on the direction of a normal vector of the rotating magnetic field (abbreviated to the direction of the rotating magnetic field) and on the direction of the magnetic field forming the rotating magnetic field; and a setting circuit 29 which sets the function of the function button 8c. The display device 7 is connected to the data processing circuit 26 and displays the image which is picked up by the image pick-up element 14 and is processed by the data processing circuit 26 via the radio circuits 22 and 25. The display device 7 is further connected to the control circuit 27 to display the current state of the rotating magnetic field and the state of the function setting.

The control circuit 27 receives instructing signals corresponding to the operation from the direction input device 8a, the speed input device 8b, and the function button 8c which form the operating input device 8, and executes the control operation in accordance with the instructing signals.

The control circuit 27 is connected to the storing circuit 28, and always stores, into the storing circuit 28, information on the direction of the rotating magnetic field and the direction of the magnetic field which are generated by the rotating magnetic field generating device 4 via the magnetic field control device 5. After that, even if an operation for changing the direction of the rotating magnetic field and the direction of the magnetic field is performed, the direction of the rotating magnetic field and the direction of the magnetic field continuously change for the purpose of the smooth change. Incidentally, the control circuit 27 may include the storing circuit 28.

The magnetic field control device 5 connected to the control circuit 27 generates AC current, and has an AC current generating and control unit 31 comprising three AC current generating and control circuits for generating AC current and controlling the frequency and the phase and a driver unit 32 comprising three drivers for amplifying the AC current. Output current of the three drivers are supplied to three electromagnets 33a to 33c forming the rotating magnetic field generating device 4.

The three electromagnets 33a to 33c comprise a pair of air-core coils facing each other, respectively, and are substantially orthogonal to each other. A uniform magnetic field is generated in the space between the facing coils and therefore the magnetic field is generated in the arbitrary direction with the above-mentioned structure. Preferably, the facing coils form Helmholtz coils.

In this case, referring to Fig. 2, the electromagnets 33a to 33c are arranged to generate the magnetic field in three orthogonal-axial directions. Preferably, the electromagnets 33a to 33c comprise a pair of air-core coils facing each other, respectively, and are arranged orthogonally to each other. A uniform magnetic field is generated in the space between the facing coils and therefore the magnetic field is generated in the arbitrary direction with the above-mentioned structure. More preferably, the facing coils form Helmholtz coils.

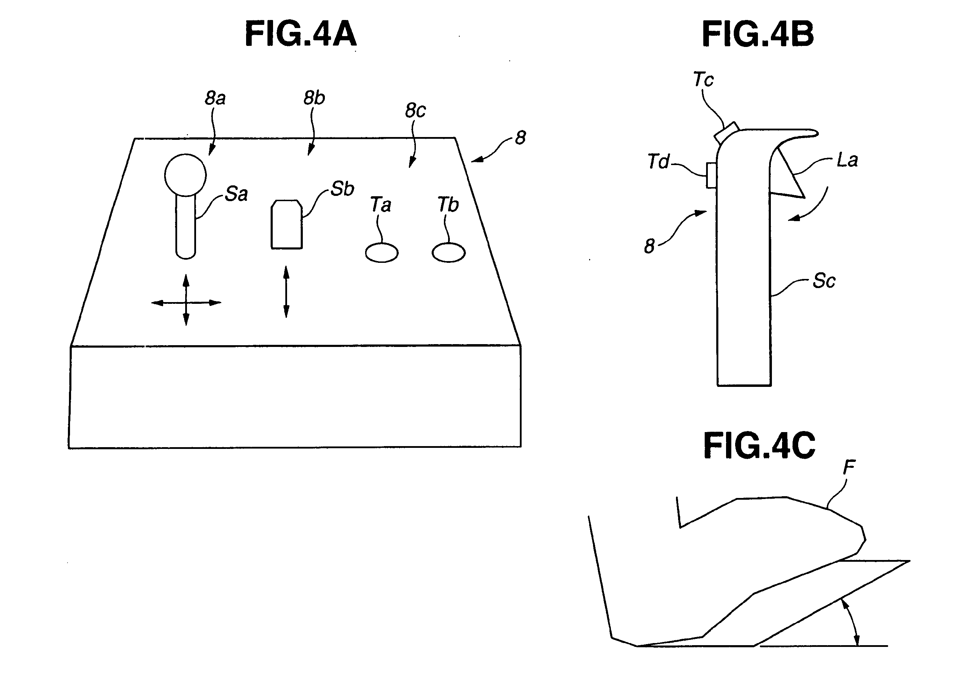

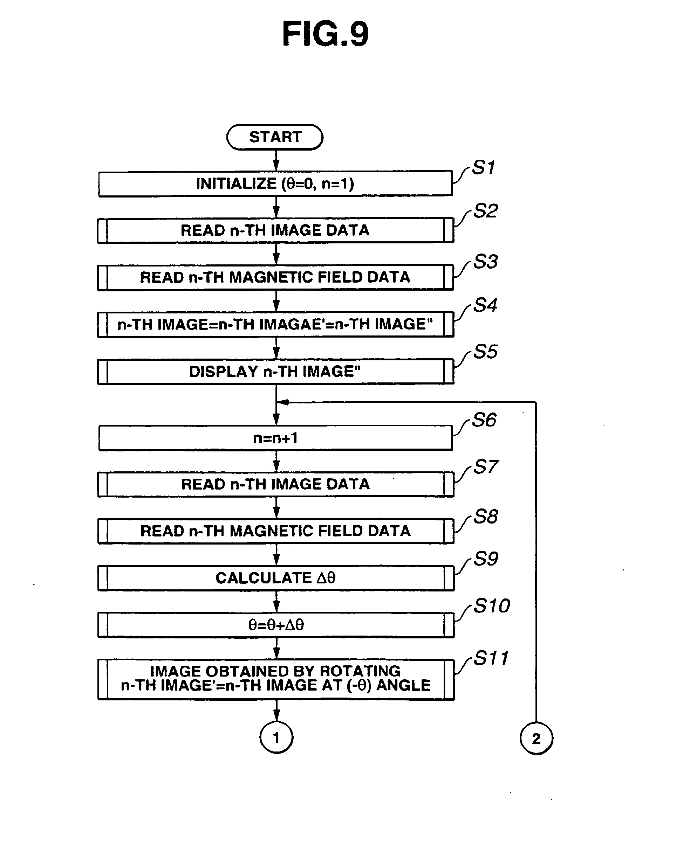

Referring to Fig. 4A, the instructing signal for the magnetic field direction is generated by operating the direction input device 8a forming the operation input device 8, the instructing signal for the rotating magnetic field of the rotating frequency is generated corresponding to the operation of speed input device 8b, and the de-centered rotating magnetic field is generated (refer to Fig. 9) by operating the function button 8c.

Specifically, the operation input device 8 comprises the direction input device 8a comprising a joystick Sa projected in the up direction of the top surface of an operating case, the speed input device 8b comprising a stick Sb, and the function button 8c comprising, for example, two buttons Ta and Tb.

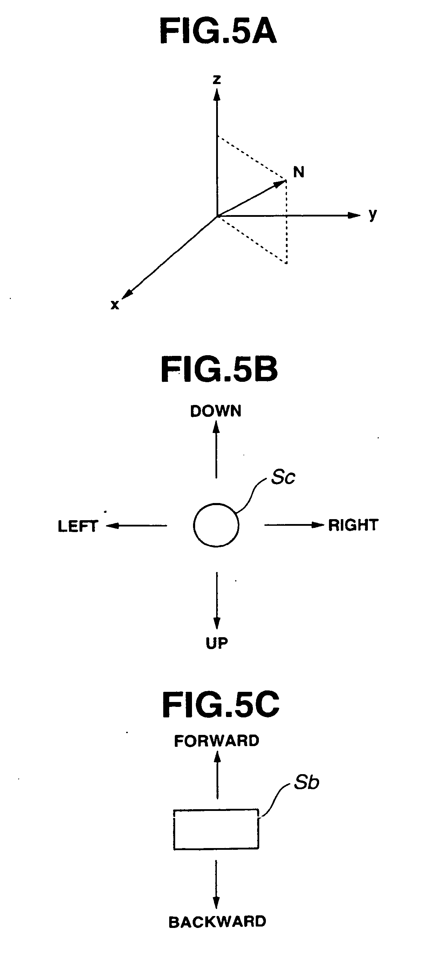

Referring to Fig. 5A, the orthogonal coordinate system is set and the direction of a normal vector N of the rotating surface of the rotating magnetic field is indicated. Then, the direction of the normal vector N is the advancing direction of the capsule main body 3, and is set by inclining the joystick Sa.

In this case, referring to Fig. 5B, the joystick Sa is inclined in the forward, backward, left, and right directions, thereby changing the advancing direction to the down, up, left, and right sides. The inclination amount in this case corresponds to the speed of angle change. By inclining the joystick Sa in the middle direction (e.g., lower left direction or upper right direction), the advancing direction of the joystick Sa is changed in the corresponding direction.

Referring to Fig. 5C, by inclining the stick Sb to the forward and backward sides, the rotating direction is set to the forward and backward sides, and the rotating frequency is changed at the inclination angle.

By the button Ta, the instructing signal for starting the de-center of the generated rotating magnetic field is generated so as to de-center the direction of the rotating magnetic field (that is, conically change the direction of the rotating magnetic field to de-center the direction of the rotating magnetic field from one direction by a de-centering angle) and, then, the magnet 16 included in the capsule main body 3 starts so-called jiggling (rotation so that the spindle of a spinning top is jiggled).

Therefore, the button Ta functions as the instructing signal for starting the jiggling, and the button Tb generates the instructing signal for stopping the de-center of the rotating magnetic field, that is, instructing signal for stopping the jiggling. A function of the setting circuit 29 presets the strength of magnetic field, a value of an angle (angle φ, which will be described later) in the case of instructing the jiggling and the setting of the frequency for jiggling. This setting may arbitrarily be changed while checking the display device 7 by the operator.

Referring to Fig. 4B, according to one modification, the operation input device 8 shown in Fig. 4A may have a lever La which can be inclined to the top of a joystick Sc and which changes the rotating speed of the capsule main body 3 by changing the rotating frequency of the rotating magnetic field depending on the amount of inclination, a button Tc which instructs the rotating direction of the rotating magnetic field by the ON/OFF operation, and a function button Td (in the case of one function button Td, having a function for switching from OFF to ON and for switching from ON to OFF if the function button Td is ON) as the de-centering function of the rotating magnetic field.

Then, the operation input device 8 can be operated by the one hand and the operability is improved as compared with the case of both-hand operation as shown in Fig. 4A.

Referring to Fig. 4A, a foot switch F shown in Fig. 4C may be used in place of the stick Sb and the rotating frequency may be changed by the pressing amount. In addition to the joystick and the foot switch, the operation input unit 8 may comprise a personal computer and the operation may be performed by using a mouse, keyboard, and GUI (Graphical User Interface).

Further, in this case, the operability is improved by displaying the GUI on the display device 7. For example, a cursor is placed on the image obtained by the capsule main body and the cursor instructs the desired direction of the capsule main body, thereby instructing the advancing direction of the capsule.

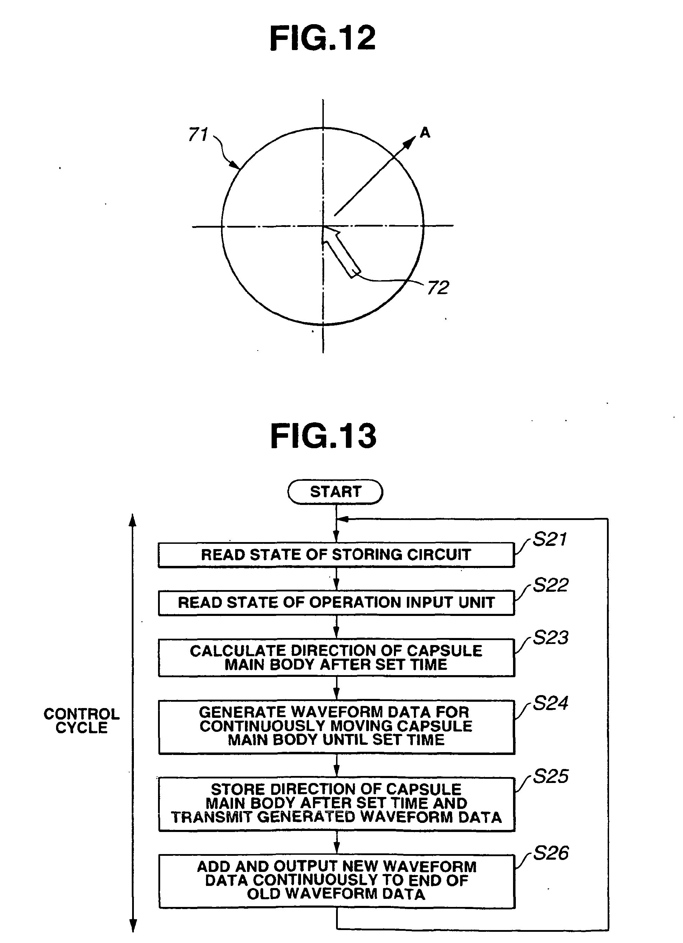

In this case, the distance from the center of the image to the cursor corresponds to the speed for changing the direction of the capsule main body and thus the operability is further improved. This operation will be described with reference to Fig. 12. Fig. 12 shows an image 71 obtained by the capsule main body, arranging a cursor 72 in the center of the image 71.

Then, when the direction of the capsule main body is changed to a direction A by the operator as shown in Fig. 12, the cursor 72 is moved in the direction A by a mouse (not shown), thereby generating the similar signal upon operating the joystick Sc by a personal computer (not shown) and transmitting the generated signal to the control circuit 27.

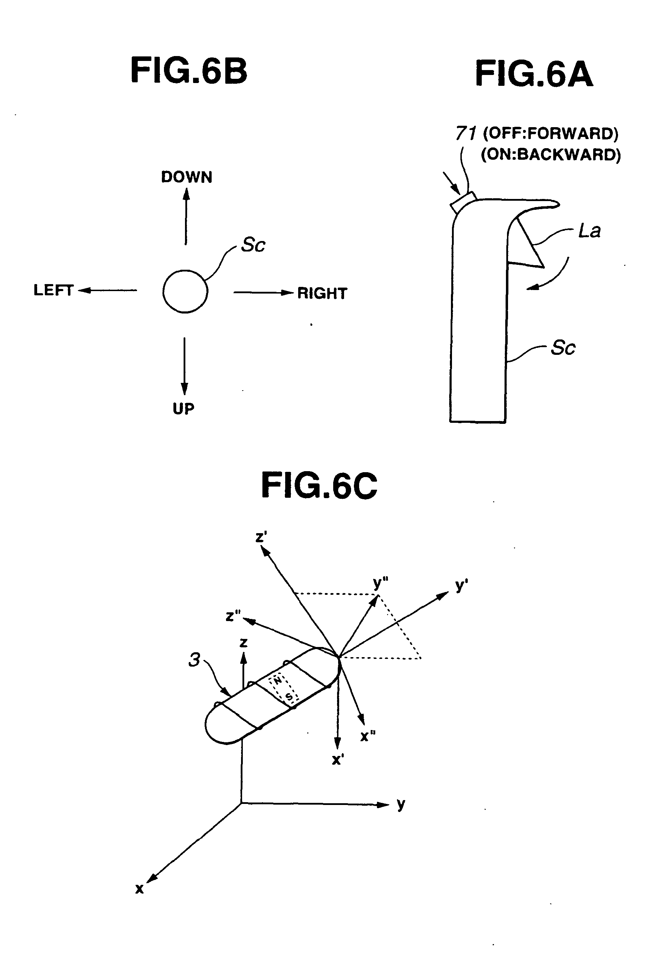

Fig. 6 shows explanatory diagrams of the operating functions in the case of using the joystick Sc shown in Fig. 4B. Fig. 6A shows an example of the structure excluding the function button Td shown in Fig. 4B, Fig. 6B shows a function for changing the advancing direction by inclining the joystick Sc, and Fig. 6C shows an explanatory diagram of the operation for actually changing the advancing direction of the capsule main body 3.

In this case, Fig. 6B shows the function for changing the direction for generating the rotating magnetic field by the inclination of the joystick Sc shown in Fig. 6A and for further changing the moving direction of the capsule main body 3. According to the first embodiment, referring to Fig. 6B (or Fig. 5B), the direction for generating the rotating magnetic field is controlled so that the capsule main body 3 moves in the direction for inclining the joystick Sc.

Further, the direction for generating the rotating magnetic field (inverse rotation of the advancing) is controlled by changing the rotating frequency with the inclination amount of the lever La so that the capsule main body moves forward when the button Tc is OFF and it moves backward when it is ON.

Referring to Fig. 6B, in order to smoothly change the advancing direction, the state of the capsule main body 3 or state of the rotating magnetic field always needs to be grasped. According to the first embodiment, the state of the rotating magnetic field (specifically, the direction of the rotating magnetic field and direction of the magnetic field) is always stored in the storing circuit 28.

Specifically, the control circuit 27 receives the operation instructing signal in the operation input unit 8 as first operation-input-means shown in Fig. 1, and outputs the control signal for generating the rotating magnetic field corresponding to the instructing signal to the magnetic field control device 5. Further, the control circuit 27 stores the information on the direction of the rotating magnetic field and on the direction of the magnetic field into the storing circuit 28.

The specific operation will be described with reference to a flowchart shown in Fig. 13. Referring to Fig. 13, the ordinate indicates the time passage. Steps S21 to S25 indicate the operation steps of the control circuit 27. First, in step S21, the control circuit 27 reads the state of the storing circuit 28.

Next, the control circuit 27 reads the state of the operation input unit 8 (S22). The control circuit 27 calculates the direction after the set time of the capsule main body based on the state of the storing circuit 28 and the state of the operation input unit 8 (S23). After calculation, the control circuit 27 generates waveform data serving as a control signal for continuously operating the capsule main body until the set time (S24). The control circuit 27 records the direction of the capsule main body after the set time to the storing circuit 28 and transmits the generated waveform data to the AC current generating and control unit 31 (S25).

The AC current generating and control unit 31 continuously adds the (new) waveform data transmitted from step S25 to the end of old waveform data transmitted at the previous time, and outputs, to the rotating magnetic field generating device 4, as the waveform data for driving the generation of the magnetic field via the driver unit 32 (S26). Incidentally, in the initial processing, the processing in step S26 is null because the old waveform data transmitted at the previous time is absent. The waveform data is inputted to the AC current generating and control unit 31 and, then, the data is continuously added to the end of the already-inputted waveform data.

After the processing in step S26, the control circuit 27 returns to the processing in step S21. As mentioned above, the processing in the closed loop in steps S21 to S26 is repeated at the predetermined control cycle. The control circuit 27 continuously outputs (magnetically induces) the waveform data for controlling the generation of the rotating magnetic field, and simultaneously changes the direction of the capsule main body in real time. Since the control cycle in this case is one sec or less (preferably, not more than 100 mS), the capsule main body is smoothly guided.

Therefore, the storing circuit 28 continuously stores information on the rotating magnetic field generated by the rotating magnetic field generating device 4 and on the direction of the magnetic field which forms the rotating magnetic field and which periodically changes.

The present invention is not limited to the case in which the storing circuit 28 stores the information corresponding to the control signal for the rotating magnetic field and the direction of the magnetic field from the control circuit 27. Further, the magnetic field control device 5 side may transmit information which determines the direction of the rotating magnetic field and direction of the magnetic field actually outputted to the rotating magnetic field generating device 4 via the AC current generating and control unit 31 and the driver unit 32 in the magnetic field control device 5, based on the control signal outputted from the control circuit 27 to the magnetic field control device 5, and the information may be stored in the storing circuit 28.

Upon starting or stopping the application of the rotating magnetic field and in the case of changing the direction of the rotating magnetic field (in other words, the advancing direction of the capsule main body) according to the first embodiment, the rotating magnetic field is controlled so that it is continuously changed without the operation of sharp force to the capsule main body 3, but with the smooth operation.

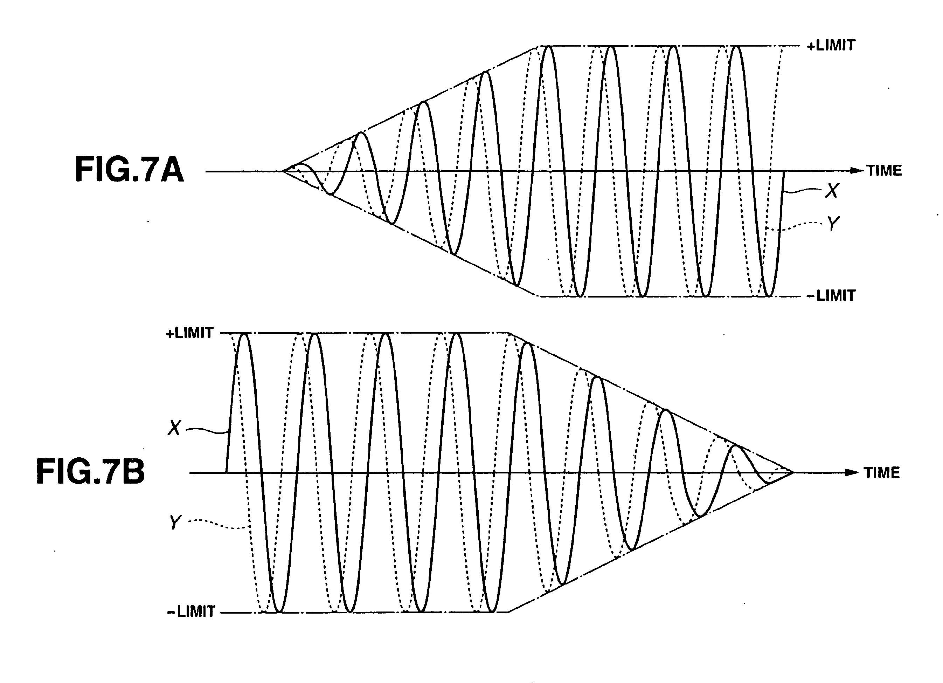

Specifically, the generating direction of the rotating magnetic field is the Z direction. Further, reference symbols Hx and Hy (abbreviated to X and Y for the purpose of brief drawings of Fig. 7) denote magnetic field components generated by the rotating magnetic field generating device 4 along the X and Y directions on the plane vertical to the Z direction so as to generate the rotating magnetic field. Then, upon starting the application of the rotating magnetic field, referring to Fig. 7A, the strength of the rotating magnetic field is continuously increased. Upon stopping the application of the rotating magnetic field, referring to Fig. 7B, the strength of the rotating magnetic field is continuously reduced.

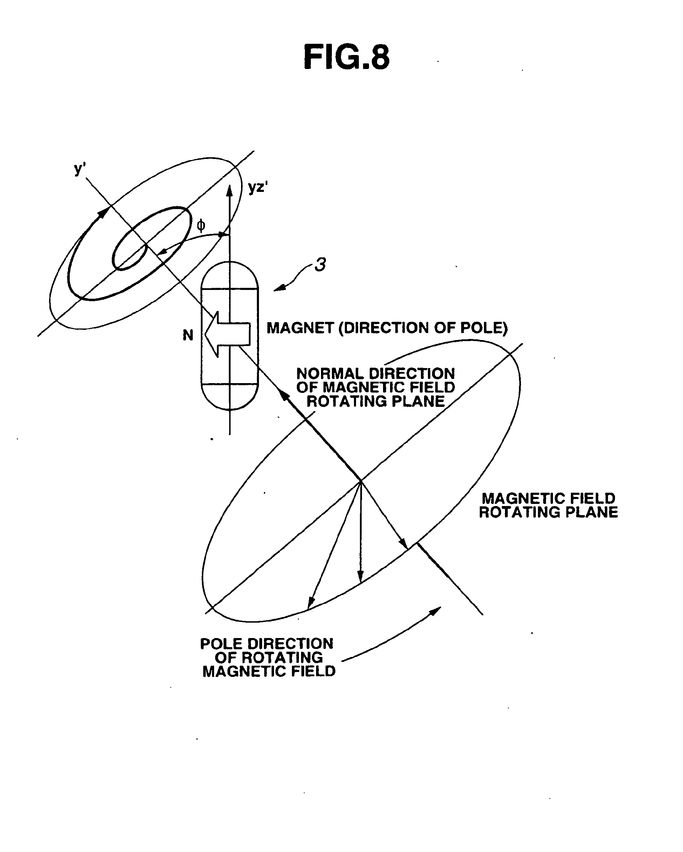

Fig. 8 shows the state for applying the rotating magnetic field, and further shows that the level of the rotating magnetic field continuously increases from the zero level upon applying the rotating magnetic field to the capsule main body 3.

The above-mentioned control operation enables the smooth maintaining of the operation of the capsule main body 3 even upon starting the application of the rotating magnetic field and upon stopping the application of the rotating magnetic field.

Preferably, not only the strength of the rotating magnetic field but also the rotating frequency of the rotating magnetic field continuously changes upon starting to applying the rotating magnetic field. Specifically, upon starting to apply the rotating magnetic field, the frequency of the rotating magnetic field is controlled so that it gradually increases. Thus, since the rotating speed of the capsule main body is gradually increased, the rotation of the capsule main body smoothly starts. Further, upon stopping the application of the rotating magnetic field, the frequency of the rotating magnetic field is controlled so that it gradually reduces. Consequently, since the rotating speed of the capsule main body gradually reduces, the rotation of the capsule main body smoothly stops.

Of course, both the frequency and the strength of magnetic field may continuously change.

According to the first embodiment, upon guiding the capsule main body 3 as the medical apparatus main body by using the rotating magnetic field, when storing, in the storing circuit 28, the information on the current state of the rotating magnetic field for determining the advancing direction of the capsule main body 3 and changing the advancing direction, the rotating magnetic field is controlled so that it continuously changes to advance the capsule main body 3 in the next advancing direction by referring to the information stored in the storing circuit 28. Thus, the guiding operation of the medical main body can be natural.

The operation with the above-mentioned structure will be described according to the first embodiment.

In the case of examining the body cavity by using the capsule main body 3, the patient swallows the capsule main body 3. When the capsule main body 3 inserted in the body cavity passes through the esophagus, the illumination is performed with the illuminating element 15, the image picked up by the image pick-up element 14 is transmitted by radio waves to the extracorporeal processing device 6 via the radio circuit 22.

In the processing device 6, the radio circuit 25 receives image data and the data processing circuit 26 stores demodulated image data in an image storing device (such as a hard disk), performs the display processing, and outputs the processed data to the display device 7, thereby displaying the images sequentially picked up by the capsule main body 3.

The operator can estimate the current schematic position of the capsule main body 3 based on the image displayed on the display device 7. When it is determined that the image of the esophagus is picked up now and the portion as an examination target is on the deeper side such as the small intestine, it is better to advance the portion on the route more speedy. In this case, the initial setting is performed that the direction of the rotating magnetic field generated in the rotating magnetic field generating device 4 (direction of the normal direction) is on the bottom along the body height of the patient. The spiral projection 12 arranged to the capsule main body 3 in this case is formed like a right screw while the direction of the field of view for the image pick-up operation using the image pick-up element 14 is on the front side.

Upon first operating the direction input device 8a so as to generate the rotating magnetic field, the storing circuit 28 does not store the just-before information corresponding to the rotating magnetic field, therefore, the control circuit 27 initializes the setting circuit 29, the setting screen for the initial setting is displayed on the display device 7, and the direction of the rotating magnetic field generated in the initial setting is selected and set by the operator. The operator first performs the instructing operation for setting the generating direction of the rotating magnetic field to the bottom side along the body height of the patient, thereby storing initial generating information of the rotating magnetic field into the storing circuit 28.

The operator previously sets the level of the rotating magnetic field (level (amplitude) of the rotating magnetic field on the magnetic field rotating plane shown in Fig. 8) by using the setting circuit 29, and further sets the prevention of generation of the rotating magnetic field so that the level of the rotating magnetic field is not less than the set level. The setting information of the setting circuit 29 is stored in the storing circuit 28. The operator sets the maximum rotating frequency of the rotating magnetic field and the maximum level of the speed for changing the direction of the capsule main body, as mentioned above.

Then, the stick Sb shown in Fig. 4A or button Tc shown in Fig. 4B in the operation input device 8 is turned off and the lever La is turned over. Thus, the control circuit 27 reads the information stored in the storing circuit 28 and controls so as to generate the rotating magnetic field in the bottom side along the body height of the patient. That is, the rotating magnetic field generating device 4 generates the rotating magnetic field via the magnetic field control device 5 based on the information read from the storing circuit 28.

In this case, when the down side along the body height of the patient is in the Z direction, the rotating magnetic field generating device 4 continuously increases the magnetic field component forming the generating rotating magnetic field from the null state as shown by the X and Y directions in Fig. 7A. When the magnetic field component reaches a predetermined level (+Limit and -Limit in Fig. 7), the rotating magnetic field generating device 4 maintains the amplitude thereof.

When the lever La is inclined, the rotating magnetic field is generated with the frequency corresponding to the amount of inclining operation. Fig. 7 shows a state in which the level (amplitude) of the rotating magnetic field upon the generation (application) thereof is changed and then reaches a predetermined rotating magnetic field in accordance with the operation for inclining the lever La at a certain angle for the purpose of a brief drawing. When the lever La is inclined further, the rotating magnetic field has a short period, that is, a high frequency.

Here, upon starting and stopping the application of the rotating magnetic filed, the frequency may gradually change so as to prevent the sharp change in rotating frequency of the capsule main body. Alternatively, both the amplitude and the frequency may gradually change.

By extracorporeally applying the rotating magnetic field, magnetic torque operates to the magnet 16 included in the capsule main body 3 inserted in the body cavity and then the capsule main body 3 rotates. The capsule main body 3 can advance fast so that the screw rotates in the state in which the spiral projection 12 arranged to the outer peripheral surface of the capsule main body 3 is contact with the inner wall in the body cavity.

The lever La is released and the operation of the lever La stops. Then, the lever La returns to the center position (with the amount 0 of operation). Referring to Fig. 7B, the components of the rotating magnetic field are continuously reduced to zero. That is, by continuously changing the rotating magnetic field even upon stopping the application of the rotating magnetic field, the operation of the capsule main body 3 is smoothly or naturally controlled.

The storing circuit 28 always stores the information on the state of the rotating magnetic field (direction of the rotating magnetic field and direction of the magnetic field), and further stores the information on the state of the rotating magnetic field while the lever La is handed off and the application of the rotating magnetic field stops.

In the next operation for applying the rotating magnetic field again, the rotating magnetic field similar to that in the case of stopping the application of the rotating magnetic field is generated based on the information stored in the storing circuit 28. Of course, in this case, the rotating magnetic field is controlled so that it continuously increases as shown in Fig. 7A.

According to the first embodiment, upon starting or stopping the application of the rotating magnetic field, the level of the rotating magnetic field continuously changes and consequently the force acting on the capsule main body 3 continuously changes upon applying or stopping the rotating magnetic field. The capsule main body 3 can smoothly advance by applying the rotating magnetic field with the higher speed, and it can be guided to the target portion side in a short time.

The moving speed of the capsule main body 3 increases by applying the rotating magnetic field in the esophagus as mentioned above. However, when the capsule main body 3 moves from the stomach to the duodenum, the rotating magnetic field may be applied.

In this case, it is confirmed based on the picked-up image that the capsule main body 3 enters the duodenum from the stomach. The capsule main body 3 can move faster by applying the rotating magnetic field in the direction of the duodenum. In this case, when the direction of the duodenum is the Z axis direction, referring to Fig. 7A, the rotating magnetic field is applied. Upon stopping the application of the rotating magnetic field, the rotating magnetic field changes as shown in Fig. 7B.

General cases are described. Referring to Fig. 6C, the capsule main body 3 exists in the three-dimensional space, and the front side (field-of-view direction side) of the capsule main body 3 in the longitudinal direction is the y' direction (referring to Fig. 6C, the orthogonal coordinate system (x', y', z') is set so that the front side of the capsule main body 3 in the longitudinal direction is the y' direction). In order to advance the capsule main body 3 in the y' direction by the rotating magnetic field, the direction of the rotating magnetic field applied (in the normal direction) is set to the y' direction and then the rotating magnetic field is applied.

Referring to Fig. 7A, upon applying the rotating magnetic field, the magnetic field component forming the rotating magnetic field continuously increasingly changes. That is, as shown by a thick and spiral line on the top in Fig. 8, the rotating magnetic field gradually increases.

Referring to Fig. 6C, when the advancing direction of the capsule main body 3 changes from the y' direction to the y" direction on the top thereof (referring to Fig. 6C, the orthogonal coordinate system (x", y", z") is set in which the front side of the capsule main body 3 in the longitudinal direction is the y" direction), the direction input device 8a is operated. For example, by inclining the joystick Sa or stick Sc to the hand side, the direction of the rotating magnetic field is changed to the y" direction as the top side of the y' direction.

In this case, the stick Sc is inclined to the hand side and the lever La is inclined, thereby continuously changing the rotating magnetic field. Further, the advancing direction of the capsule main body 3 smoothly or naturally changes by applying the rotating magnetic field.

The rotating magnetic field is controlled by the control circuit 27. Specifically, referring to Fig. 7A, the rotating magnetic field is basically generated by referring to the input information from the direction input device 8a such as the stick Sc based on the information on the rotating magnetic field in the y' direction stored in the storing circuit 28 so that the y" direction becomes the direction of the rotating magnetic field (incidentally, referring to Fig. 7A, the advancing direction is the Z direction and therefore the magnetic field component is different from that shown in Fig. 7A).

When the lever La is still inclined and then the stick Sc is inclined to the hand side, the control circuit 27 controls the direction of the rotating magnetic field so that it changes continuously to the y" direction from the y' direction.

Specifically, the control circuit 27 calculates the direction (y" direction) of the capsule main body after a predetermined time (corresponding to the control cycle as described above) based on the information on the rotating magnetic field stored in the storing circuit 28 and the operation information of the direction input device 8a. Next, the control circuit 27 calculates the waveform of the rotating magnetic field for continuously changing the direction of the capsule from the y' direction to the y" direction so that the capsule main body 3 smoothly moves. The control circuit 27 transmits the calculated waveform data to the AC current generating and control unit 31. Thus, the continuously changed waveform controls the electromagnets 33a to 33c, and the direction of the rotating magnetic field smoothly and naturally changes in the y" direction. Therefore, the direction of the capsule main body smoothly and naturally changes in the y" direction.

As mentioned above, according to the first embodiment, in the case of changing the advancing direction of the capsule main body 3, the rotating magnetic field continuously changes by referring to the information on the rotating magnetic field stored in the storing circuit 28. Consequently, the advancing direction of the capsule main body 3 can smoothly change.

According to the first embodiment, the operator operates the function button 8c, thereby generating the so-called jiggling rotating magnetic field so that the direction of the rotating magnetic field is periodically de-centered. The information on the de-centered angle preset by the setting circuit 29 is stored in the storing circuit 28, the function button 8c is operated, then, the control circuit 27 reads the information on the de-centered angle, and the rotating magnetic field is generated so that the direction of the rotating magnetic field is de-centered by the de-centered angle.

When the capsule main body 3 exists in the luminal portion having the diameter larger than the maximum outer diameter thereof including the spiral projection 12, only a part of the spiral projection 12 is contact with the inner wall of the luminal portion and the capsule main body 3 does not smoothly move by the normal rotating magnetic field.

In this case, the operator generates the magnetic field having the de-centered direction of the rotating magnetic field (hereinafter, referred to a jiggling magnetic field), thereby jiggling the capsule main body 3 with the juggling magnetic field. Thus, the outer diameter of the capsule main body 3 is substantially (virtually) increased upon the jiggling operation and the spiral projection 12 is in contact with the inner wall of the luminal portion. As compared with the normal rotating magnetic field, the capsule main body 3 efficiently advances with the smoothness and stability.

For example, with reference to Fig. 8, referring to Fig. 8, when the y' direction is the advancing direction of the capsule main body 3, the button Ta (or Td) of the function button 8c is operated and then the control circuit 27 controls the rotating magnetic field in the direction of the button Ta (or Td) so that the jiggling magnetic field is generated at an angle $\phi$ de-centered from the direction. Referring to Fig. 8, the direction yz' forming the angle $\phi$ to the y' direction varies with time. In this case, the angle $\phi$ is formed between the direction yz' and the y' direction (the angle gradually increases from the smaller angle until it reaches the angle $\phi$ as follows).

Upon generating the jiggling magnetic field, the angle gradually increases from the level 0 of the rotating magnetic field in the y' direction, that is, the jiggling magnetic field is generated at the angle which gradually increases from that at the small angle. Then, when the angle reaches the angle $\phi$, the jiggling magnetic field maintains.

Like the operation just before the spinning top falls, the capsule main body 3 changes to a state in which the core of the capsule main body 3 gradually and increasingly jiggles from the small rotating jiggle state, that is, the capsule main body 3 continuously changes from the jiggling at the small angle to the jiggling at the large angle, when it enters the jiggling state with the predetermined angle $\phi$, and the capsule main body 3 maintains the state.

When the direction input device 8 comprises a PC or the like, various parameters of the jiggling can arbitrarily be set.

As a result of the jiggling operation, the capsule main body 3 can advance while the luminal portion having the inner diameter larger than the outer diameter of the capsule main body 3 is stable. Further, as the result of the jiggling, the image pick-up range is substantially wide and the image of the inner wall of the luminal portion can widely be picked up.

By operating the button Tb for stopping the jiggling operation in the function button 8c, on the contrary to the foregoing, the state changes from the jiggling magnetic field at the angle $\phi$ to the jiggling magnetic field at the gradually reduced angle. Further, the level of the magnetic field gradually reduces.

As mentioned above, according to the fist embodiment, since the jiggling magnetic field is generated, the magnetic field can stably be generated without manually jiggling or "shaking" operation of operating means corresponding to the direction input operating device 8a so as to conventionally generate the jiggling magnetic field, and the operability is remarkably improved.

In the above description, the jiggling magnetic field is generated by operating the function button 8c after stopping the rotating magnetic field. However, when the function button 8c is operated during applying the rotating magnetic field, the angle is gradually increased from the state of the rotating magnetic field and the jiggling magnetic field is generated to maintain the state at the angle $\phi$. In such a state, the button for stopping the jiggling is operated and then the opposite operation is executed.

According to the first embodiment, the rotation of the capsule main body 3 results in the rotation of the image picked up by the image pick-up element 14. Therefore, if the image in this state is displayed on the display device 7, the displayed image becomes the rotated image and the operability for instructing the desired direction by using the direction input device 8b is reduced. The rotation of the displayed image needs to be stopped.

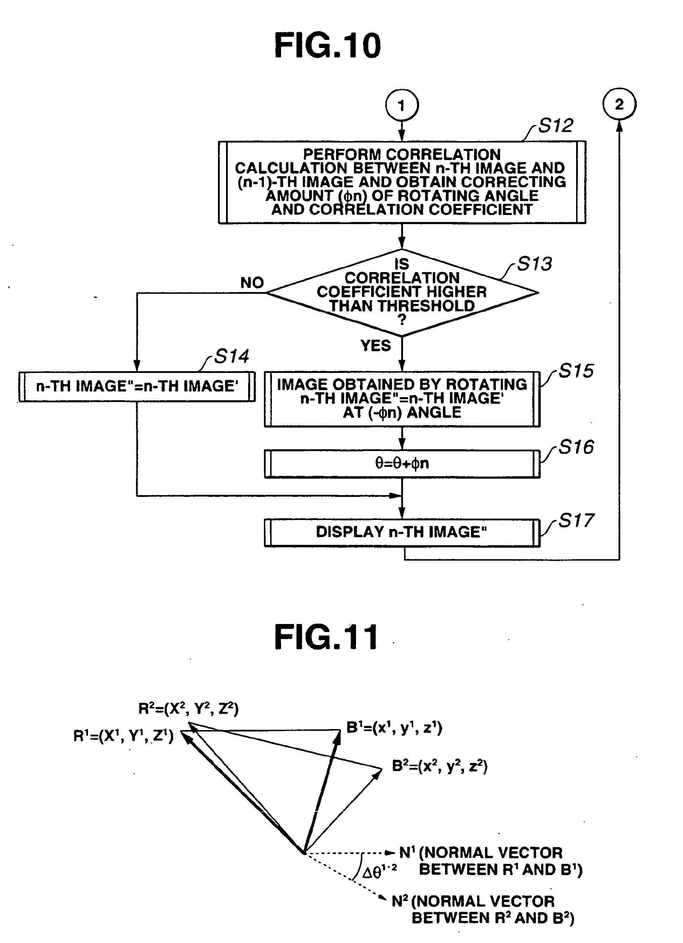

According to the present embodiment, the rotated image is corrected to the image in the rotating stop state, that is, the processing shown in Figs. 9 and 10 is performed, as will be described later (described in detail in Japanese Patent Application No. 2002-105493).

The capsule main body 3 sequentially picks up the images on time series and stores digital video signals in the memory 21. Under the control of the control circuit 27 in the processing device 6, the digital video signals are stored as image data in an inner memory of the data processing circuit 26 via the radio circuits 22 and 25. In this case, the control circuit 27 in the processing device 6 stores magnetic field data containing the direction of the rotating magnetic field and the direction of the magnetic field upon picking up the image data, correlated with the image data stored in the inner memory.

The inner memory sequentially stores a plurality of pieces of image data containing first image data, second image data, ..., n-th image data and further stores a plurality of pieces of the magnetic field data correlated with the image data, containing first magnetic field data, second magnetic field data, ..., and n-th magnetic field data.

Referring to Fig. 9, in step S1, the control circuit 27 in the processing device 6 initializes parameters $\theta$ (rotating angle of the total images) and n (image number) and sets $\theta = 0$ and n = 1. In step S2, the control circuit 27 reads the n-th image data (the first image data in this case) stored in the inner memory. In step S3, the n-th magnetic field data (first magnetic field data in this case) containing the direction of the rotating magnetic field and the direction of the magnetic field in this time is read from the inner memory.

Then, in step S4, the control circuit 27 sets the n-th image data' as the first corrected image data and the n-th image data" as the second corrected image data to image data equal to the n-th image data. In step S5, the control circuit 27 controls the data processing circuit 26, and displays the displayed image based on the n-th image data" on the display device 7.

In step S6, the control circuit 27 increments n by 1. In step S7, the control circuit 27 reads the n-th image data (second image data in this case) stored in the inner memory. In step S8, the control circuit 27 reads, from the inner memory, the n-th magnetic field data (second magnetic field data in this case) containing the direction of the rotating magnetic field and the direction of the magnetic field. In step S9, the control circuit 27 calculates the rotating angle $\Delta\theta$ of the n-th image and the (n-1)-th image. Specifically, referring to Fig. 11, $B^1(x^1, y^1, z^1)$ represents the direction of the rotating magnetic field of the first magnetic field data as the magnetic field data of the first image data, $R^1(X^1, Y^1, Z^1)$ represents the normal direction of the rotating magnetic field, $B^2(x^2, y^2, z^2)$ represents the direction of the rotating magnetic field of the second magnetic field data as the magnetic field data of the second image data, and $R^2(X^2, Y^2, Z^2)$ represents the normal direction of the rotating magnetic field.

The advancing direction of the capsule main body 3 gradually changes. If the angle between $B^1$ and $B^2$ is simply the rotating angle, the actual rotating angles cannot match. Then, in consideration of the change in advancing direction of the capsule main body 3 for the rotating angle, referring to Fig. 11, the rotating angle $\Delta\theta$ represents the angle formed between the normal vector $N^1$ of $R^1$ and $B^1$ and the normal vector $N^2$ of $R^2$ and $B^2$.

The rotating angle $\Delta\theta$ is obtained as follows.

$$N^1 = (y^1 Z^1 - Y^1 z^1,\ z^1 X^1 - Z^1 x^1,\ x^1 Y^1 - X^1 y^1)$$

$$N^2 = (y^2 Z^2 - Y^2 z^2,\ z^2 X^2 - Z^2 x^2,\ x^2 Y^2 - X^2 y^2)$$

The normal vectors $N^1$ and $N^2$ are unit vectors and therefore the rotating angle $\Delta\theta$ is calculated as follows.

$$\Delta\theta^{1\cdot2} = \cos^{-1}\{(y^1z^1 - Y^1z^1)(y^2z^2 - Y^2z^2)\}$$

As time passes, the rotating angle can be calculated by sequentially obtaining $\Delta\theta^{1\cdot2}$, $\Delta\theta^{2\cdot3}$, ...$\Delta\theta^{(n-2)\cdot(n-1)}$, and $\Delta\theta^{(n-1)\cdot n}$.

The total rotating angle $\theta$ is the sum of the above angles and is represented by $\theta = \Sigma\Delta\theta^{(k-1)\cdot k}$. Therefore, in step S10, the control circuit 27 sets ($\theta = \theta + \Delta\theta$) to the total rotating angle. For example, the second image becomes the image which is obtained by rotating the first image in the direction in the drawing at (the rotating angle $\theta$ + error). The error is a rotating-angle error between the rotating angle of the capsule main body 3 caused by the load of the rotation of the body wall and the spiral projection 12 of the capsule main body 3 and the rotating angle of the magnetic field forming the rotating magnetic field.

In step S11, the control circuit 27 sets the n-th image data' as the first corrected image data to the image data which is obtained by rotating the n-th image data by the angle ($-\theta$). Thus, for example, the second image' is obtained, serving as the first corrected image without considering the error.

Next, the processing routine shifts to step S12 in Fig. 10. In step S12, the control circuit 27 executes the well-known correlation calculation of the n-th image data and the (n-1) image data, and obtains the correlation coefficient with the rotating angle correcting amount ($\phi$n). In step S13, the control circuit 27 determines whether or not the correlation coefficient is higher than a predetermined threshold. As the result of determination, it is determined whether or not the rotating angle error is ignored.

If the correlation coefficient is not higher than the predetermined threshold, in step S14, the control circuit 27 sets the n-th image data" as the second corrected image data to the n-th image data' as the first corrected image data and the processing routine shifts to step S17. If the correlation coefficient is not higher than the predetermined threshold, that is, when the image sharply changes, the result of the correlation processing is not used. Then, at the timing of performing the processing in step S11 (setting the n-th image data' as the first corrected image data to the image data obtained by rotating the n-th image data by the angle ($-\theta$)), the correction for the image rotation completes.

If the correlation coefficient is higher than the predetermined threshold, in step S15, the control circuit 27 sets the image data obtained by rotating, by the angle ($-\phi$n), the n-th image data" as the second corrected image data = the n-th image data' as the first corrected image data. Thus, for example, the second image" as the second corrected image is obtained. In step S16, the total rotating angle $\theta$ is set to ($\theta + \phi$n) and the processing routine shifts to step S17.

In step S17, the control circuit 27 controls the image processing circuit 32 and displays, on the display device 5, the displayed image for which the correction for rotation is completed based no the n-th image data". The processing routine returns to step S6 in Fig. 9.

The image displayed on the display device 7 has a circular contour. Thus, the image rotation processing is displayed without the user's consciousness.

When the driving frequency of the capsule is approximately equal to the frequency for obtaining and displaying the capsule image, the image is not theoretically rotated and therefore the step of correcting the image rotation as mentioned above may be omitted.

Further, in this case, the image displayed on the display device 7 may not be circular. If it is rectangular, square, or octagonal, the pixel of the image pick-up element is effectively used and displayed.

According to the first embodiment, upon applying the rotating magnetic field and stopping the application thereof and upon changing the direction of the rotating magnetic field, the rotating magnetic field continuously changes and therefore the operation of the capsule main body 3 such as movement is smoothly performed.

In place of swallowing the capsule main body 3, after inserting the capsule like a suppository in the rectum from the anus of the patient, the image pick-up element may magnetically be induced so as to examine the portion from the ileum such as the large intestine or small intestine to the jejunum.

(Second embodiment)

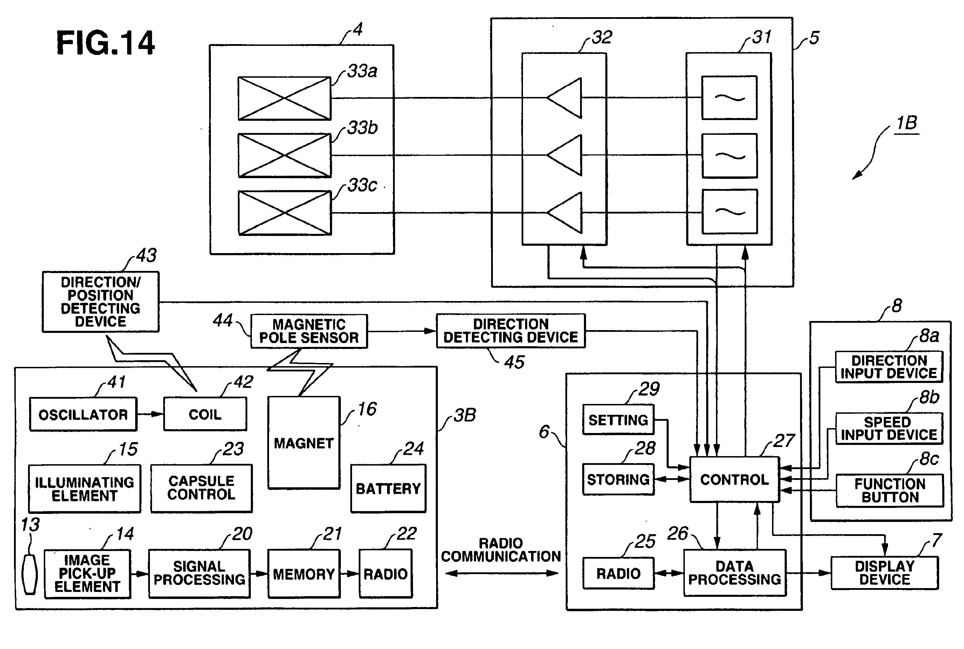

Next, the second embodiment of the present disclosure, according to the invention will be described with reference to Figs. 14 to 17.

Referring to Fig. 14, a capsule medical apparatus guiding system 1B according to the second embodiment comprises a capsule main body 3B comprising an oscillator 41 and a coil 42 for generating an alternating magnetic field around it by an output signal from the oscillator 41 which are arranged in the capsule main body 3 in the capsule medical apparatus guiding system 1 shown in Fig. 1.

The capsule main body 3B externally has: a direction/position detecting device 43 which detects the direction of the capsule main body 3B in the longitudinal direction based on the alternating magnetic field from the coil 42 and further detects the position thereof; a magnetic pole sensor 44 which detects the direction of the magnet 16 included in the capsule main body 3B; and a (magnet) direction detecting device 45 which detects the direction of the magnet based on the output from the magnetic pole sensor 44.

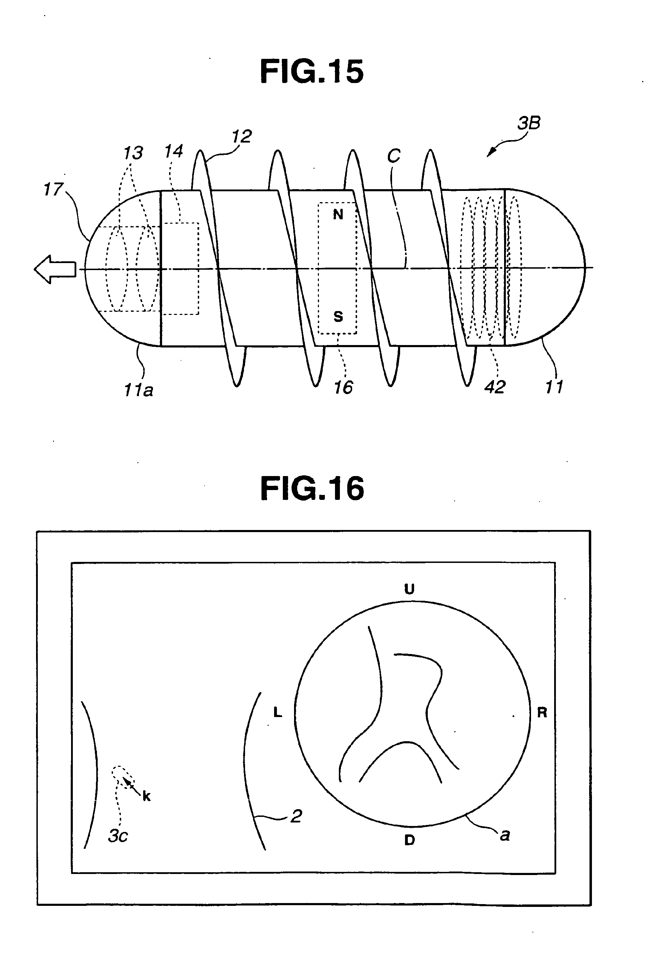

Fig. 15 shows the capsule main body 3B according to the second embodiment. Referring to Fig. 15, the capsule main body 3B is formed so that the coil 42 is accommodated in the capsule main body 3 shown in Fig. 3A near the rear end of the exterior container 11 to be wound in a predetermined direction set to the longitudinal direction of the capsule main body 3B, specifically, like solenoid. The direction/position detecting device 43 comprises a plurality of sense coils for detecting the alternating magnetic field and detects the direction and the position of the coil 42 based on the signals detected by the sense coils. The magnetic pole sensor 44 comprises a plurality of magnetic pole sensors 44 and detects the direction of the magnetic field pole of the magnet 16 based on output signals from the plurality of magnetic pole sensors. Further, the direction such as the front side of the capsule main body 3 in the longitudinal direction can be detected based on the arrangement of the magnet 16 and the coil 42 arranged in the capsule main body 3.

In place of the coil 42, an antenna may be used, then, radio waves radiated by the antenna are received by the direction/position detecting device 43, and the direction and position in the longitudinal direction of the capsule main body 3B are detected.

The information detected by the direction/position detecting device 43 and the direction detecting device 45 is inputted to the control circuit 27 in the processing device 6.

When the operation input device 8 is operated, the control circuit 27 generates the rotating magnetic field and controls the direction of the generated rotating magnetic field based on the information stored in the storing circuit 28 and the information detected by the direction/position detecting device 43 and the direction detecting device 45.

According to the second embodiment, upon displaying the image picked up by the capsule main body 3B on the display device 7, the image is displayed as shown in Fig. 16.

That is, an image display area a on the right of the display screen displays the image picked up by the capsule main body 3B. The left side of the display screen displays a schematic body shape 2 of the patient. At the schematic position in the body shape 2 where the capsule main body 3B is detected, an image 3c indicating the outer shape is displayed together with a directional cursor k indicating the front direction of the capsule main body 3B in the longitudinal direction.

Similarly to the first embodiment, the image display area a displays the image picked up by the image pick-up element 14 while the forward side of the image pick-up element 14 is in the up direction. In this case, the rotation of the capsule main body 3B is corrected and displayed according to the method described according to the first embodiment. However, according to the second embodiment, the direction detecting device 45 detects the direction of the magnet 16, therefore, the direction for displaying the image is determined by using the detected output, the image rotation processing is performed, and the image is displayed as shown in Fig. 16.

According to the second embodiment, with the above-mentioned structure, it is possible to detect the direction of the capsule main body 3B (including both the longitudinal direction and the vector direction at which the edge cover 11a is on the front side) and the direction of the magnetic pole of the magnet 16. Basically, when the information on the rotating magnetic field in the storing circuit 28 is not used and the operation is inputted such as changing the direction of the capsule main body 3B, the capsule main body 3B smoothly changes in the instructed direction.

Therefore, according to the second embodiment, those are combined, thereby selecting and operating an operation mode from a plurality of modes which are previously set by the setting circuit 29.

Hereinbelow, typical operation modes will be described.

In a first mode, means for counting time by a timer (not shown) is provided. A basis time of a time interval relatively shorter set by the setting circuit 29 is set as the basis, the direction of the rotating magnetic field is changed at the time interval shorter than the basis time, and the application of the rotating magnetic field is instructed again after stopping the application of the rotating magnetic field. Then, the control circuit 27 performs the control operation in accordance with the instruction input based on the information stored in the storing circuit 28.

That is, upon issuing the instruction for changing the direction of the rotating magnetic field within the short time interval, the state does not nearly change from the information stored in the storing circuit 28 just before it. Therefore, the error is small without using the information on the capsule main body 3B detected by the direction/position detecting device 43 and the same operation and advantages as those according to the first embodiment are obtained.

Upon issuing the instruction for changing the direction of the rotating magnetic field after the time interval longer than the time interval of the basis time, the direction of the capsule main body 3B might greatly be changed. The control circuit 27 controls the operation so that the advancing direction of the capsule main body 3B smoothly changes by using the information on the direction and position of the capsule main body 3B detected by the direction/position detecting device 43 and the information on the direction of the magnet 16 detected by the direction detecting device 45.

Upon changing the advancing direction (thrust generating direction) of the capsule main body 3B, as described above according to the first embodiment, the application or updating of the rotating magnetic field is continuously changed, thereby smoothly changing the advancing direction of the capsule main body 3B.

In the first mode, as mentioned above, the detecting means of the direction of the capsule main body 3B is provided. When the rotating magnetic field is applied again after the rotating magnetic field to the capsule main body 3B is stopped and a long time passes and when the direction of the capsule main body 3B changes after the rotating magnetic field is stopped and then a long time passes, the proper rotating magnetic field is applied based on the information detected by the detecting means, such as the direction of the capsule main body 3B, and the capsule main body 3B can smoothly advance and the direction thereof may smoothly change.

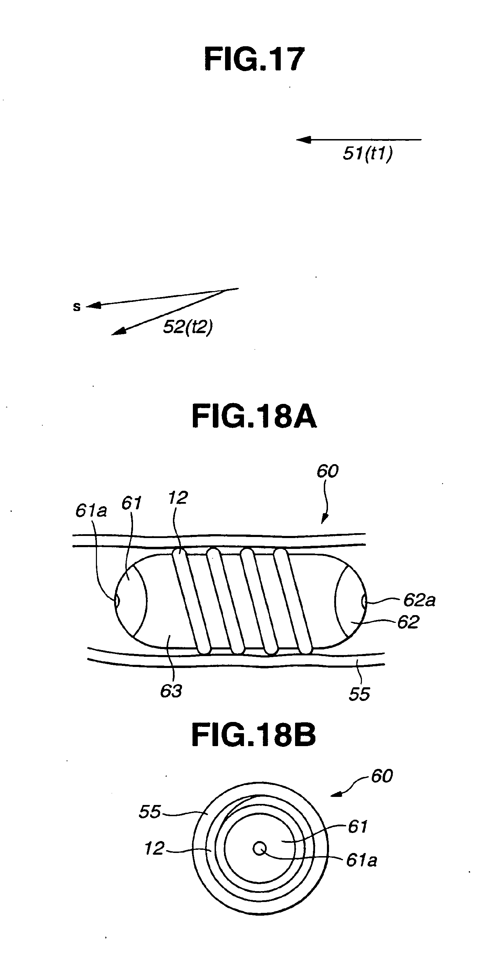

The operation in this case will briefly be described with reference to Fig. 17. Referring to Fig. 17, the position of the capsule main body 3B is indicated by a vector 51 (t1) at a time t1 at which the application of the rotating magnetic field to the capsule main body 3B is stopped. At a time t2 after a certain time passes from the time t1, the capsule main body 3B shifts to a vector 52 (t2).

When the operation of the direction input device 8a enables the instruction input for applying the rotating magnetic field which advances the capsule main body 3B in an advancing direction s at the time t2, the control circuit 27 smoothly guides the capsule main body 3B by continuously changing the rotating magnetic field in the direction corresponding to that of the capsule main body 3B at the time t2 to the rotating magnetic field in the advancing direction s (not the rotating magnetic field in the direction corresponding to that of the capsule main body 3B at the time tl) based on the information detected at the time t2 (or time just before it).

In a second mode, the storing circuit 28 sequentially stores the information detected by the direction/position detecting device 43 and the direction detecting device 45, and updates the previously-stored information. Further, when the operation input device 8 performs the operation input, the control circuit 27 implements the operation corresponding to the operation input based on the information stored in the storing circuit 28.

In this case, the information stored in the storing circuit 28 reflects the state of the capsule main body 3B practically in real time. The information is stored in the storing circuit 28 so as to correct the direction in the neutral state from the direction input device 8a corresponding to the state of the capsule main body and thus the operability is improved.

The operation result in this mode is almost the same as the operation result described in the first mode. However, the operation is controlled so that the state corresponding to the current state of the capsule main body 3B is changed to the state corresponding to the operation input (it is not necessary to consider the state change of the capsule main body 3B during the time for no magnetic inducing) and therefore the operability is improved.

According to the second embodiment, if the state of the capsule main body 3B is changed during the time for no magnetic inducing, the magnetic inducing is smoothly and stably performed.

According to the second embodiment, the means for detecting the direction of the capsule main body 3B is not limited to the means using the alternating magnetic field generated by the coil 42. The direction of the capsule main body 3B may be detected by a fluoroscopic apparatus or it may be detected by using ultrasonic waves from an ultrasonic diagnostic apparatus.

In the foregoing, the direction of the capsule main body 3B and the direction of the magnetic pole of the magnet 16 are detected. In the case of continuously changing the rotating magnetic field, the information on the direction of the magnetic pole of the magnet 16 is not always necessary (for example, referring to Fig. 7A, when continuously changing (the component magnetic field of) the rotating magnetic field to be large from zero, the operation force is changed to gradually be increased from zero at the timing for applying the rotating magnetic field no matter in which direction the magnetic pole faces and therefore the information on the direction of the magnetic pole of the magnet 16 is not necessary).

In the case of changing the direction of the capsule main body 3B, it is possible to recognize the direction of the magnet 16 arranged to the capsule main body 3B by the direction of the magnetic field during applying the rotating magnetic field. Thus, the information on the magnetic pole sensor 44 is not necessary. In the case of moving the capsule main body 3B while changing the direction thereof from the still state, the information on the magnetic pole sensor 44 is not necessary by starting the capsule main body 3B as shown in Fig. 7A.

Further, the information on the direction of the capsule main body 3B from the direction/position detecting device 43 is recognized by the direction of the rotating magnetic field during applying the rotating magnetic field. Therefore, the operation of the direction/position detecting device 43 may be interval operation in which the OFF operation is performed during applying the rotating magnetic field and in another case, the ON operation is performed.

In the description, the medical apparatus main body is the capsule main body 3 or 3B comprising the capsule endoscope including the image pick-up element 14. However, the capsule medical apparatus main body may be used as a drug spray one for the cure or treatment as shown in Fig. 18. That is, in a capsule medical apparatus 60, a capsule main body 63 having the spiral projection 12 on the outer peripheral surface has a drug accommodating unit 61. The drug accommodating unit 61 has a drug spreading opening portion 61a arranged on the edge side so as to spread the drug accommodated in the drug accommodating unit 61. Fig. 18 shows the capsule medical apparatus 60 in the small intestine 55.

Further, the capsule medical apparatus 60 can extract the body fluid.

That is, the capsule medical apparatus 60 has a body fluid injecting opening portion 62a so as to extract the body fluid in a body fluid accommodating unit 62 in the capsule main body 63. The opening and closing of the opening portions 61a and 62a are performed under the communication control of the processing device 6. Therefore, an input device (not shown) such as a keyboard for instruction operation is connected to the control circuit 27 in the processing device 6. By operating the input device, a control signal is transmitted to the capsule medical apparatus 60 so as to control the opening and closing operation of the opening portions 61a and 62a.

Thus, the capsule medical apparatus 60 can discharge and spread the drug in the drug accommodating unit 61 at the target portion from the drug spreading opening portion 61a. Further, the capsule medical apparatus 60 can extract the body fluid from the body fluid injecting opening portion 62a in the body fluid accommodating unit 62.

Of course, the drug accommodating unit 61 may accommodate the drug, a hemostatic agent for stopping the bleeding, a magnetic fluid or a fluorescer that is safe to the living body for determining the bleeding portion from the outside, so as to spread them at the target portion.

The capsule medical apparatus 60 may mix the drug in the drug accommodating unit 61 to the body fluid extracted from the body fluid injecting opening portion 62a, discharge it from the drug spreading opening portion 61a, and spread it. The capsule medical apparatus 60 matches the center of gravity with the center axis of the capsule main body 63 in the longitudinal direction.

The magnetic field generated by the external magnetic field generating means is used as the rotation driving means for rotating the capsule medical apparatus (hereinafter, referred to a capsule) in the foregoing. However, the present invention is not limited to this and another rotation driving means may be used.

As the means for rotating the capsule, a dielectric member (for polarization such as a capacitor) is arranged to the capsule and thus the electric field is externally rotated and applied, thereby rotating the capsule.

In the case of not the capsule medical apparatus, but a medical apparatus with a shaft, a thick winding flexible shaft used for the ultrasonic probe is rotatable in the shaft, and a motor on the hand side is rotated, thereby rotating and advancing the capsule.

In this case, the mutual operation of the magnetic force may change the advancing direction of the medical apparatus. The direction of the medical apparatus may be changed by arranging a bending mechanism to the shaft portion. An actuator may be arranged to operate a bending mechanism for the jiggling, and the jiggling operation may be performed by repeating the bending mechanism.

The medical apparatus according to the present invention is not limited to the capsule medical apparatus and can widely be applied to medical apparatuses having an inserting portion which is inserted in the body cavity.

In place of including the battery, energy may extracorporeally be supplied by microwaves and magnetic force to drive circuits in the capsule or power may extracorporeally be supplied by a cable.

Industrial Applicability

The patient swallows the capsule main body having its outer shape that is almost cylindrical and further having a spiral projection on its outer circumferential surface. Then, the rotating magnetic field is externally applied. As a consequence, the capsule main body in the body cavity is rotated and, simultaneously, is smoothly advanced so as to smoothly obtain the living body information and perform a treatment.

**Claims**

1.  A medical apparatus guiding system (1) comprising:

    a medical apparatus main body (3) having an approximately cylindrical shape and configured to be inserted into a body cavity, the medical apparatus main body (3) being configured to generate a thrust by a spiral structure portion (12) provided on a side surface of the approximately cylindrical shape being rotated around a center axis of the approximately cylindrical shape;
    a magnet (16) provided to the medical apparatus main body (3) and arranged with a magnetic pole direction oriented toward a direction substantially perpendicular to the center axis;
    a magnetic field generating device (4) configured to generate a rotating magnetic field to be exerted on the magnet (16);
    an information providing unit comprising at least one of a storing unit (28) configured to store at least one of a state of the rotating magnetic field generated from the magnetic field generating device (4) and an advancing direction of the medical apparatus main body (3), a direction detecting unit (45) configured to detect the direction of the medical apparatus main body (3), and a magnetic pole detecting unit (44) configured to detect the direction of the magnetic pole of the magnet (16);
    an input unit (8) configured to input a change amount of a thrust generating direction of the medical apparatus main body (3); and
    a control unit (31) configured to change a thrust generating state of the medical apparatus main body (3) based on information from the information providing unit,
**characterized by**
an image pick-up element (14) provided to the medical apparatus main body (3) and configured to pick-up an image inside the body cavity;
a display device (5) configured to display an image picked-up by the image pick-up element (14);
an interface configured to allocate an operating direction of the input unit (8) to the up, down, right, and left sides of the image displayed on the display device (5); and
an image rotation correcting unit configured to perform processing for cancelling rotation of the image generated upon rotating the medical apparatus main body (3) by the rotating magnetic field generated from the magnetic field generating device (4) and cause the display device (5) to display the image on which the processing is performed.

2. The medical apparatus guiding system (1) according to Claim 1, wherein the control unit (31) is configured to continuously change the thrust generating state of the medical apparatus main body (3).

3. The medical apparatus guiding system (1) according to Claim 1, wherein
the control unit (31) is configured to control a state for generating the magnetic field of the magnetic field generating device (4) based on information from the information providing unit and the input unit (8), to thereby change the thrust generating direction of the medical apparatus main body (3).

4. The medical apparatus guiding system (1) according to Claim 1, wherein the input unit (8) is configured to further input the thrust generating amount of the medical apparatus main body (3).

5. The medical apparatus guiding system (1) according to Claims 1 or 3, wherein the input unit (8) has an automatic return mechanism configured to automatically return the operation amount to zero upon stopping the operation.

6. The medical apparatus guiding system (1) according to Claims 1 or 3, wherein the medical apparatus main body (3) further includes a medication system or a body fluid extracting system.

7. The medical apparatus guiding system (1) according to Claim 1, wherein the medical apparatus main body is a capsule endoscope.

8. The medical apparatus guiding system (1) according to Claims 1 or 3, wherein the input unit (8) is configured to further input a rotating frequency of the rotating magnetic field generated from the magnetic field generating device (4).

9. The medical apparatus guiding system (1) according to Claims 1 or 3, wherein the input unit (8) is configured to further input a rotating direction of the rotating magnetic field generated from the magnetic field generating device (4).

10. The medical apparatus guiding system (1) according to Claims 1 or 3, wherein the input unit (8) is configured to further input the change amount of the direction of the rotating magnetic field generated from the magnetic field generating device (4).

11. The medical apparatus guiding system (1) according to Claims 1 or 3, wherein when the rotating frequency of the medical apparatus main body (3) is changed, the control unit (31) is configured to continuously change the rotating frequency of the rotating magnetic field generated from the magnetic field generating device (4).

12. The medical apparatus guiding system (1) according to Claims 1 or 3, wherein when the strength of the rotating magnetic field generated from the magnetic field generating device (4) is changed, the control unit (31) is configured to control strength of the rotating magnetic field generated from the magnetic field generating device (4) so that it is continuously changed.

13. The medical apparatus guiding system (1) according to Claims 1 or 3, wherein the magnet (16) is arranged approximately at the position of the center of gravity in the medical apparatus main body (3).

14. The medical apparatus guiding system (1) according to Claims 1 or 3, wherein the magnet (16) is arranged near the end portion of the medical apparatus main body (3).

15. The medical apparatus guiding system (1) according to Claims 1 or 3, wherein the information providing unit comprises at least two or more of the storing unit (28), the direction detecting unit (45), and the magnetic pole detecting unit (44), and the control unit (31) is configured to change the information providing unit referred to based on the control history.

**16.** The medical apparatus guiding system (1) according to Claim 3, wherein the control unit (31) is configured to continuously change the thrust generating state of the medical apparatus main body (3).

**17.** A control method of a medical apparatus guiding system (1) according to claim 1,
the control method comprising
a step for changing a thrust generating state of the medical apparatus main body (3), based on information from the information providing unit,
**characterized by**
a step for performing an image rotation correcting processing for cancelling rotation of the image generated upon rotating the medical apparatus main body (3) by the rotating magnetic field generated from the magnetic field generating device (4); and
a step for causing the display device (5) to display the image on which the image rotation correcting processing is performed.

**Patentansprüche**

**1.** Führungssystem für eine medizinische Vorrichtung (1) aufweisend:

einen Hauptkörper einer medizinischen Vorrichtung (3) mit einer annähernd zylindrischen Form und dazu eingerichtet, in einen Körperhohlraum eingeführt zu werden, wobei der Hauptkörper der medizinischen Vorrichtung (3) dazu eingerichtet ist, durch einen Teil mit einer Spiralstruktur (12), die an einer Seitenfläche der annähernd zylindrischen Form bereitgestellt ist, die um eine Mittelachse der annähernd zylindrischen Form rotiert, eine Axialbewegung zu erzeugen;
einen Magneten (16), der an dem Hauptkörper der medizinischen Vorrichtung (3) bereitgestellt ist und mit einer Magnetpolrichtung orientiert zu einer Richtung im Wesentlichen senkrecht zu der Mittelachse angeordnet ist;
eine Vorrichtung zum Erzeugen eines Magnetfelds (4), die dazu eingerichtet ist, ein sich drehendes Magnetfeld zu erzeugen, welches auf den Magneten (6) angewendet wird;
eine Informationsbereitstellungseinheit aufweisend eine Speichereinheit (28), die dazu eingerichtet ist, einen Zustand des sich drehenden Magnetfelds, welches durch die Vorrichtung zum Erzeugen eines Magnetfelds (4) erzeugt wurde, und/oder eine Fortbewegungsrichtung des Hauptkörpers der medizinischen Vorrichtung (3) zu speichern, eine Richtungserfassungseinheit (45), die dazu eingerichtet ist, die Richtung des Hauptkörpers der medizinischen Vorrichtung (3) zu erfassen und/oder eine Magnetpolerfassungseinheit (44), die dazu eingerichtet ist, die Richtung des Magnetpols des Magneten (16) zu erfassen;
eine Eingabeeinheit (8), die dazu eingerichtet ist, einen Änderungsbetrag einer Axialbewegungserzeugungsrichtung des Hauptkörpers der medizinischen Vorrichtung (3) einzugeben;
eine Steuerungseinheit (31), die dazu eingerichtet ist, basierend auf Information der Informationsbereitstellungseinheit einen Axialrichtungserzeugungszustand des Hauptkörpers der medizinischen Vorrichtung (3) zu ändern,
**gekennzeichnet durch**
ein Bildaufnahmeelement (14), das an dem Hauptkörper der medizinischen Vorrichtung (3) bereitgestellt und dazu eingerichtet ist, ein Bild in der Körperhöhle aufzunehmen;
eine Anzeigevorrichtung (5), die dazu eingerichtet ist, ein **durch** das Bildaufnahmeelement (14) aufgenommene Bild anzuzeigen;
eine Schnittstelle, die dazu eingerichtet ist, den auf der Anzeigevorrichtung (5) angezeigten oberen, unteren, rechten und linken Seiten des Bildes eine Betriebsrichtung der Eingabeeinheit (8) zuzuordnen; und
eine Bilddrehungskorrektureinheit, die dazu eingerichtet ist, eine Verarbeitung zum Beenden des Drehens des Bildes, das erzeugt wurde, nach dem Drehen des Hauptkörpers der medizinischen Vorrichtung (3) **durch** das sich drehende Magnetfeld, welches von der Vorrichtung zum Erzeugen eines Magnetfelds (4) erzeugt wurde, auszuführen, und die Anzeigevorrichtung (5) veranlasst, dass Bild anzuzeigen, an dem die Verarbeitung ausgeführt wurde.

**2.** Führungssystem für eine medizinische Vorrichtung (1) nach Anspruch 1, bei dem die Steuerungseinheit (31) dazu eingerichtet ist, fortlaufend den Axialbewegungserzeugungszustand des Hauptkörpers der medizinischen Vorrichtung (3) zu ändern.

**3.** Führungssystem für eine medizinische Vorrichtung (1) nach Anspruch 1, bei dem die Steuerungseinheit (31) dazu eingerichtet ist, einen Zustand zum Erzeugen des Magnetfelds der Vorrichtung zum Erzeugen eines Magnetfelds (4) basierend auf Information der Informationsbereitstellungseinheit und der Eingabeeinheit (8) zu steuern, um dadurch die Axialbewegungserzeugungsrichtung des Hauptkörpers der medizinischen Vorrichtung (3) zu ändern.

**4.** Führungssystem für eine medizinische Vorrichtung (1) nach Anspruch 1, bei dem die Eingabeeinheit (8) dazu eingerichtet ist, ferner den Axialbewegungserzeugungsbetrag des Hauptkörpers der medizinischen Vorrichtung (3) einzugeben.

**5.** Führungssystem für eine medizinische Vorrichtung (1) nach Anspruch 1 oder 3, bei dem die Eingabeeinheit (8) einen automatischen Rückkehrmechanismus aufweist, der dazu eingerichtet ist, bei Beendigung der Betätigung automatisch den Betätigungsbetrag auf null zurückzusetzen.

**6.** Führungssystem für eine medizinische Vorrichtung (1) nach Anspruch 1 oder 3, bei dem der Hauptköper der medizinischen Vorrichtung (3) ferner ein Arzneimittelsystem oder ein Körperflüssigkeitsextraktionssystem enthält.

**7.** Führungssystem für eine medizinische Vorrichtung (1) nach Anspruch 1, bei dem der Hauptkörper der medizinischen Vorrichtung ein Kapselendoskop ist.

**8.** Führungssystem für eine medizinische Vorrichtung (1) nach Anspruch 1 oder 3, bei dem die Eingabeeinheit (8) dazu eingerichtet ist, ferner eine Drehfrequenz des sich drehenden magnetischen Felds, das durch die Vorrichtung zum Erzeugen eines Magnetfelds (4) erzeugt wurde, einzugeben.

**9.** Führungssystem für eine medizinische Vorrichtung (1) nach Anspruch 1 oder 3, bei dem die Eingabeeinheit (8) dazu eingerichtet ist, ferner eine Drehrichtung des sich drehenden magnetischen Felds, das durch die Vorrichtung zum Erzeugen eines Magnetfelds (4) erzeugt wurde, einzugeben.

**10.** Führungssystem für eine medizinische Vorrichtung (1) nach Anspruch 1 oder 3, bei dem die Eingabeeinheit (8) dazu eingerichtet ist, ferner den Änderungsbetrag der Richtung des sich drehenden magnetischen Felds, das durch die Vorrichtung zum Erzeugen eines Magnetfelds (4) erzeugt wurde, einzugeben.

**11.** Führungssystem für eine medizinische Vorrichtung (1) nach Anspruch 1 oder 3, bei dem, wenn die Drehfrequenz des Hauptkörpers der medizinischen Vorrichtung (3) geändert wird, die Steuerungseinheit (31) dazu eingerichtet ist, fortlaufend die Drehfrequenz des sich drehenden magnetischen Felds, das durch die Vorrichtung zum Erzeugen eines Magnetfelds (4) erzeugt wurde, zu ändern.

**12.** Führungssystem für eine medizinische Vorrichtung (1) nach Anspruch 1 oder 3, bei dem, wenn die Stärke des durch die Vorrichtung zum Erzeugen eines Magnetfelds (4) erzeugten sich drehenden magnetischen Felds geändert wird, die Steuerungseinheit (31) dazu eingerichtet ist, die Stärke des durch die Vorrichtung zum Erzeugen eines Magnetfelds (4) erzeugten sich drehenden magnetischen Felds zu steuern, so dass es sich fortlaufend ändert.

**13.** Führungssystem für eine magnetische Vorrichtung (1) nach Anspruch 1 oder 3, bei dem der Magnet (16) annähernd an einer Position des Körperschwerpunkts in dem Hauptkörper der medizinischen Vorrichtung (3) angeordnet ist.

**14.** Führungssystem für eine medizinische Vorrichtung (1) nach Anspruch 1 oder 3, bei dem der Magnet (16) in der Nähe des Endteils des Hauptkörpers der medizinischen Vorrichtung (3) angeordnet ist.

**15.** Führungssystem für eine medizinische Vorrichtung (1) nach Anspruch 1 oder 3, bei dem die Informationsbereitstellungseinheit mindestens zwei oder mehr der Speichereinheit (28), der Richtungserfassungseinheit (45) und der Magnetpolerfassungseinheit (44) aufweist, wobei die Steuerungseinheit (31) dazu eingerichtet ist, die in Bezug genommene Informationsbereitstellungseinheit basierend auf der Steuerungsvergangenheit zu ändern.

**16.** Führungssystem für eine medizinische Vorrichtung (1) nach Anspruch 3, bei dem die Steuerungseinheit (31) dazu eingerichtet ist, fortlaufend den Axialbewegungserzeugungszustand des Hauptkörpers der medizinischen Vorrichtung (3) zu ändern.

17. Steuerungsverfahren eines Führungssystems für eine medizinische Vorrichtung (1) nach Anspruch 1, wobei das Steuerungsverfahren aufweist
einen Schritt eines Änderns eines Axialbewegungserzeugungszustands des Hauptkörpers der medizinischen Vorrichtung (3) basierend auf Information der Informationsbereitstellungseinheit,
**gekennzeichnet durch**
einen Schritt eines Ausführens einer Bilddrehungskorrekturverarbeitung zum Beenden einer Drehung des Bildes, das erzeugt wurde nach dem Drehen des Hauptkörpers der medizinischen Vorrichtung (3) **durch** das von der Vorrichtung zum Erzeugen eines Magnetfelds (4) erzeugte sich drehende magnetische Feld; und
einen Schritt eines Veranlassens der Anzeigevorrichtung (5) zum Anzeigen des Bildes, an dem die Bilddrehungskorrekturverarbeitung ausgeführt wurde.

## Revendications

1. Système (1) de guidage d'appareil médical comprenant :

   un corps principal (3) d'appareil médical ayant une forme approximativement cylindrique et configuré pour être inséré dans une cavité corporelle, le corps principal (3) d'appareil médical étant configuré pour générer une poussée par une partie (12) à structure en spirale prévue sur une surface latérale de la forme approximativement cylindrique étant tournée autour d'un axe central de la forme approximativement cylindrique ;
   un aimant (16) prévu sur le corps principal (3) d'appareil médical et agencé avec un sens de pôle magnétique orienté vers un sens sensiblement perpendiculaire à l'axe central ;
   un dispositif (4) de génération de champ magnétique configuré pour générer un champ magnétique rotatif destiné à être exercé sur l'aimant (16) ;
   une unité de délivrance d'informations comprenant au moins une parmi une unité (28) de stockage configurée pour stocker au moins un parmi un état du champ magnétique rotatif généré par le dispositif (4) de génération de champ magnétique et un sens d'avancement du corps principal (3) d'appareil médical, une unité (45) de détection de sens configurée pour détecter le sens du corps principal (3) d'appareil médical, et une unité (44) de détection de pôle magnétique configurée pour détecter le sens du pôle magnétique de l'aimant (16) ;
   une unité (8) d'entrée configurée pour entrer une quantité de changement d'un sens de génération de poussée du corps principal (3) d'appareil médical ; et
   une unité (31) de commande configurée pour changer un état de génération de poussée du corps principal (3) d'appareil médical sur la base d'informations provenant de l'unité de délivrance d'informations,
   **caractérisé par**
   un élément (14) de capture d'image prévu sur le corps principal (3) d'appareil médical et configuré pour capturer une image à l'intérieur de la cavité corporelle ;
   un dispositif (5) d'affichage configuré pour afficher une image capturée par l'élément (14) de capture d'image ;
   une interface configurée pour allouer un sens de fonctionnement de l'unité (8) d'entrée aux côtés haut, bas, droit et gauche de l'image affichée sur le dispositif (5) d'affichage ; et
   une unité de correction de rotation d'image configurée pour exécuter un traitement pour annuler une rotation de l'image générée lors d'une rotation du corps principal (3) d'appareil médical par le champ magnétique rotatif généré par le dispositif (4) de génération de champ magnétique et pour faire que le dispositif (5) d'affichage affiche l'image sur laquelle le traitement est exécuté.

2. Système (1) de guidage d'appareil médical selon la revendication 1, dans lequel l'unité (31) de commande est configurée pour changer de façon continue l'état de génération de poussée du corps principal (3) d'appareil médical.

3. Système (1) de guidage d'appareil médical selon la revendication 1, dans lequel
   l'unité (31) de commande est configurée pour commander un état de génération du champ magnétique du dispositif (4) de génération de champ magnétique sur la base d'informations provenant de l'unité de délivrance d'informations et de l'unité (8) d'entrée, pour ainsi changer le sens de génération de poussée du corps principal (3) d'appareil médical.

4. Système (1) de guidage d'appareil médical selon la revendication 1, dans lequel l'unité (8) d'entrée est configurée pour entrer en outre la quantité de génération de poussée du corps principal (3) d'appareil médical.

5. Système (1) de guidage d'appareil médical selon la revendication 1 ou 3, dans lequel l'unité (8) d'entrée a un mécanisme de retour automatique configuré pour remettre automatiquement la quantité d'opération à zéro à l'arrêt de l'opération.

**6.** Système (1) de guidage d'appareil médical selon la revendication 1 ou 3, dans lequel le corps principal (3) d'appareil médical inclut en outre un système de médication ou un système d'extraction de fluide corporel.

**7.** Système (1) de guidage d'appareil médical selon la revendication 1, dans lequel le corps principal d'appareil médical est un endoscope de type capsule.

**8.** Système (1) de guidage d'appareil médical selon la revendication 1 ou 3, dans lequel l'unité (8) d'entrée est configurée pour entrer en outre une fréquence de rotation du champ magnétique rotatif généré par le dispositif (4) de génération de champ magnétique.

**9.** Système (1) de guidage d'appareil médical selon la revendication 1 ou 3, dans lequel l'unité (8) d'entrée est configurée pour entrer en outre un sens de rotation du champ magnétique rotatif généré par le dispositif (4) de génération de champ magnétique.

**10.** Système (1) de guidage d'appareil médical selon la revendication 1 ou 3, dans lequel l'unité (8) d'entrée est configurée pour entrer en outre la quantité de changement du sens du champ magnétique rotatif généré par le dispositif (4) de génération de champ magnétique.

**11.** Système (1) de guidage d'appareil médical selon la revendication 1 ou 3, dans lequel, lorsque la fréquence de rotation du corps principal (3) d'appareil médical est changée, l'unité (31) de commande est configurée pour changer de façon continue la fréquence de rotation du champ magnétique rotatif généré par le dispositif (4) de génération de champ magnétique.

**12.** Système (1) de guidage d'appareil médical selon la revendication 1 ou 3, dans lequel, lorsque la force du champ magnétique rotatif généré par le dispositif (4) de génération de champ magnétique est changée, l'unité (31) de commande est configurée pour commander la force du champ magnétique rotatif généré par le dispositif (4) de génération de champ magnétique de façon à ce qu'elle soit changée de façon continue.

**13.** Système (1) de guidage d'appareil médical selon la revendication 1 ou 3, dans lequel l'aimant (16) est agencé approximativement à la position du centre de gravité dans le corps principal (3) d'appareil médical.

**14.** Système (1) de guidage d'appareil médical selon la revendication 1 ou 3, dans lequel l'aimant (16) est agencé à proximité de la partie d'extrémité du corps principal (3) d'appareil médical.

**15.** Système (1) de guidage d'appareil médical selon la revendication 1 ou 3, dans lequel l'unité de délivrance d'informations comprend au moins deux ou plus parmi l'unité (28) de stockage, l'unité (45) de détection de sens et l'unité (44) de détection de pôle magnétique, et l'unité (31) de commande est configurée pour changer l'unité de délivrance d'informations à laquelle il est fait référence sur la base de l'historique de commande.

**16.** Système (1) de guidage d'appareil médical selon la revendication 3, dans lequel l'unité (31) de commande est configurée pour changer de façon continue l'état de génération de poussée du corps principal (3) d'appareil médical.

**17.** Procédé de commande d'un système (1) de guidage d'appareil médical selon la revendication 1,
le procédé de commande comprenant
une étape de changement d'un état de génération de poussée du corps principal (3) d'appareil médical, sur la base d'informations provenant de l'unité de délivrance d'informations,
**caractérisé par**
une étape d'exécution d'un processus de correction de rotation d'image pour annuler une rotation de l'image générée à la rotation du corps principal (3) d'appareil médical par le champ magnétique rotatif généré par le dispositif (4) de génération de champ magnétique ; et
une étape pour faire que le dispositif (5) d'affichage affiche l'image sur laquelle le processus de correction de rotation d'image est exécuté.

FIG.1

# FIG.2

MAGNETIC FIELD CONTROL DEVICE ～5

1

8

DIRECTION INPUT DEVICE ～8a

SPEED INPUT DEVICE ～8b

FUNCTION BUTTON ～8c

4

16 12

N
S

13 14

3

RADIO COMMUNICATION

PROCESSING DEVICE ～6

DISPLAY DEVICE ～7

EP 1 591 058 B1

# FIG.3A

# FIG.3B

UP DIRECTION OF
IMAGE PICK-UP ELEMENT 14

DIRECTION
OF FIELD
OF VIEW

IMAGE PICK-UP
ELEMENT

# FIG.4A

# FIG.4B

# FIG.4C

# FIG.5A

z

N

y

x

# FIG.5B

DOWN

*Sc*

LEFT ← ○ → RIGHT

UP

# FIG.5C

FORWARD

*Sb*

BACKWARD

# FIG.6B

# FIG.6A

71 (OFF:FORWARD)
(ON:BACKWARD)

# FIG.6C

FIG.7A

FIG.7B

# FIG.8

y'

yz'

φ

3

N

MAGNET (DIRECTION OF POLE)

NORMAL DIRECTION
OF MAGNETIC FIELD
ROTATING PLANE

MAGNETIC FIELD
ROTATING PLANE

POLE DIRECTION
OF ROTATING
MAGNETIC FIELD

# FIG.9

```
              ┌─────────────┐
              │   START     │
              └──────┬──────┘
                     ▼
┌──────────────────────────────────────┐  S1
│        INITIALIZE (θ=0, n=1)          │
└──────────────────┬───────────────────┘
                   ▼
┌──────────────────────────────────────┐  S2
│        READ n-TH IMAGE DATA           │
└──────────────────┬───────────────────┘
                   ▼
┌──────────────────────────────────────┐  S3
│     READ n-TH MAGNETIC FIELD DATA     │
└──────────────────┬───────────────────┘
                   ▼
┌──────────────────────────────────────┐  S4
│ n-TH IMAGE=n-TH IMAGAE'=n-TH IMAGE"   │
└──────────────────┬───────────────────┘
                   ▼
┌──────────────────────────────────────┐  S5
│        DISPLAY n-TH IMAGE"            │
└──────────────────┬───────────────────┘
                   ▼
┌──────────────────────────────────────┐  S6
│              n=n+1                    │
└──────────────────┬───────────────────┘
                   ▼
┌──────────────────────────────────────┐  S7
│        READ n-TH IMAGE DATA           │
└──────────────────┬───────────────────┘
                   ▼
┌──────────────────────────────────────┐  S8
│     READ n-TH MAGNETIC FIELD DATA     │
└──────────────────┬───────────────────┘
                   ▼
┌──────────────────────────────────────┐  S9
│            CALCULATE Δθ               │
└──────────────────┬───────────────────┘
                   ▼
┌──────────────────────────────────────┐  S10
│              θ=θ+Δθ                   │
└──────────────────┬───────────────────┘
                   ▼
┌──────────────────────────────────────┐  S11
│      IMAGE OBTAINED BY ROTATING       │
│ n-TH IMAGE'=n-TH IMAGE AT (-θ) ANGLE  │
└──────────────────┬───────────────────┘
                   ▼
                  ( 1 )            ( 2 )
```

# FIG.10

PERFORM CORRELATION CALCULATION BETWEEN n-TH IMAGE AND (n-1)-TH IMAGE AND OBTAIN CORRECTING AMOUNT ($\phi$n) OF ROTATING ANGLE AND CORRELATION COEFFICIENT — S12

IS CORRELATION COEFFICIENT HIGHER THAN THRESHOLD ? — S13

NO

YES

n-TH IMAGE"=n-TH IMAGE' — S14

IMAGE OBTAINED BY ROTATING n-TH IMAGE"=n-TH IMAGE' AT (-$\phi$n) ANGLE — S15

$\theta=\theta+\phi$n — S16

DISPLAY n-TH IMAGE" — S17

# FIG.11

$R^2=(X^2, Y^2, Z^2)$

$R^1=(X^1, Y^1, Z^1)$

$B^1=(x^1, y^1, z^1)$

$B^2=(x^2, y^2, z^2)$

$\Delta\theta^{1,2}$

$N^1$ (NORMAL VECTOR BETWEEN $R^1$ AND $B^1$)

$N^2$ (NORMAL VECTOR BETWEEN $R^2$ AND $B^2$)

# FIG.12

# FIG.13

```
                    START

            READ STATE OF STORING CIRCUIT          S21

            READ STATE OF OPERATION INPUT UNIT     S22

            CALCULATE DIRECTION OF CAPSULE         S23
            MAIN BODY AFTER SET TIME

  CONTROL   GENERATE WAVEFORM DATA FOR             S24
  CYCLE     CONTINUOUSLY MOVING CAPSULE
            MAIN BODY UNTIL SET TIME

            STORE DIRECTION OF CAPSULE             S25
            MAIN BODY AFTER SET TIME AND
            TRANSMIT GENERATED WAVEFORM DATA

            ADD AND OUTPUT NEW WAVEFORM            S26
            DATA CONTINUOUSLY TO END OF
            OLD WAVEFORM DATA
```

# FIG.14

EP 1 591 058 B1

# FIG.15

# FIG.16

# FIG.17

*51(t1)*

s

*52(t2)*

# FIG.18A

# FIG.18B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3017770 B
- JP 2001179700 A
- JP 8322786 A
- JP 4008341 A
- JP 2002105493 A